# EUROPEAN PATENT APPLICATION

(11) **EP 4 595 966 A1**
(43) Date of publication of application: **06.08.2025**
(21) Application number: 24155023.5
(22) Date of filing: 31.01.2024
(51) Int. Cl.: A61K 31/713, A61P 35/04, C12N 15/113, A61K 31/00

(54) **USE OF NPY/Y5R INHIBITOR IN TREATING LIVER METASTASIS**

(71) Applicant: Friedrich-Alexander-Universität Erlangen-Nürnberg, 91054 Erlangen (DE)
(72) Inventor: DIETRICH, Peter, 91054 Erlangen (DE); BOSSERHOFF, Anja, 91054 Erlangen (DE); HELLERBRAND, Claus, 91054 Erlangen (DE)
(74) Representative: Wichmann, Hendrik

(57) **Abstract**

The present invention generally relates to an inhibitor (NPY/Y5R inhibitor) of NPY-5 receptor (Y5R) activation by neuropeptide Y (NPY) for use in the treatment of liver metastasis in a subject. The NPY/Y5R inhibitor is selected from at least one short interference RNA (siRNA) against NPY, at least one Y5R inhibitor, at least one anti-NPY antibody, and at least one anti-Y5R antibody.

## Description

### FIELD OF THE INVENTION

The present invention generally relates to an inhibitor (NPY/Y5R inhibitor) of NPY-5 receptor (Y5R) activation by neuropeptide Y (NPY) for use in the treatment of liver metastasis in a subject. The NPY/Y5R inhibitor is selected from at least one short interference RNA (siRNA) against NPY, at least one Y5R inhibitor, at least one anti-NPY antibody, and at least one anti-Y5R antibody.

### BACKGROUND OF THE INVENTION

Metastasis remains the major cause of cancer-related death, but its mechanisms are poorly understood (Ganesh *et al.,* 2021; Lambert *et al.,* 2017). In particular, metastasis to the liver is a common hallmark of most types of solid cancer including melanoma, colorectal cancer, pancreatic cancer and breast cancer (Tsilimigras *et al.,* 2021). Interactions between cancer cells and the microenvironment are crucial for the engraftment of liver metastases, which includes enabling tissue infiltration, organotropism, evasion of immune defenses and adaption to the liver niche (Tsilimigras *et al.,* 2021; Van den Eynden *et al.,* 2013; Gao et al., 2019; Mielgo et al., 2020).

Niche-derived cytokines, like transforming growth factor β (TGF-β) mediating stroma activation and immune evasion, and few so far identified chemokines inducing G-protein-coupled-receptor activation on cancer cells (Van den Eynden *et al.,* 2013) are considered as key players of chemotactic liver infiltration and colonization. CXCL12 is one of the few identified chemoattractive chemokines, which is expressed in the liver niche and promotes metastasis by cross-talk with CXC-receptor 4 on multiple types of disseminated cancer cells (Mortezaee, 2021). CXCL12 was also shown to interact with TGF-β in the liver microenvironment to drive colon cancer metastasis. Further identification of such niche-derived factors and interactions promoting liver colonization will enable specific therapeutic option, and is therefore desirable (Ganesh *et al.,* 2021; Lambert *et al.,* 2017; Tsilimigras *et al.,* 2021; Mortezaee, 2021).

Neuropeptide Y (NPY) and its G-protein-coupled receptor subtypes represent a highly conserved, chemokine-like system, which is involved in major cancer-related hallmarks including aging, autophagy, nutrient sensing, caloric restriction and hematopoietic stem-cell properties (Hirsch *et al.,* 2012; Cerdá-Reverter *et al.,* 2000; Dumont *et al.,* 1992; Reichmann *et al.,* 2016; Tatemoto *et al.,* 1982).

With respect to the liver, NPY has been identified as a crucial target in cirrhosis and its related complications including portal hypertension (Dietrich *et al.,* 2013; Hartl *et al.,* 2015) and hepatocellular carcinoma (Dietrich *et al.,* 2020). Moreover, numerous studies suggest that the NPY-system affects non-alcoholic fatty liver disease (NAFLD) *via* hepatic stellate cell activation, promotion of the metabolic syndrome and obesity, adipocyte-macrophage cross-talk promoting liver inflammation, and induction of hepatocytic cholesterol synthesis by activation of SREBP2-HMGCR-signaling.

Since NAFLD is the emerging epidemic of our century affecting one quarter of the global population, fatty change of the liver microenvironment was suggested to influence liver metastasis in multiple experimental and clinical studies. However, the question whether steatosis promotes or even inhibits liver metastasis in diverse types of cancer including melanoma, colorectal cancer and breast cancer remains controversial, since an equal number of studies reveal opposing results and clinical data provide evidence that the net effect is non-significant. These studies suggest that indirect, NAFLD-associated effectors depending on diverse circumstances (including co-morbidities, degree of steatosis, inflammatory activity and presence of fibrosis) as well as different experimental models, rather than fat accumulation per se, drives liver metastasis. The described CXCL12-CXCR4-chemokine-axis was shown to be deregulated in NAFLD and might represent one of such indirect effectors.

In contrast, the potential impact of the steatosis-associated NPY-system on fatty change of the metastatic liver microenvironment and associated liver colonization is completely unknown.

Two recent studies suggested that NPY might affect bone metastasis in Ewing sarcoma, however its potential impact on liver metastasis remained completely elusive (Hong et al., 2015; Lu et al., 2022).

### SUMMARY OF THE INVENTION

As shown in the examples section herein, the inventors now surprisingly found that hepatocyte-derived NPY is a major chemoattractive ligand promoting metastatic liver colonization. Specifically, niche-derived NPY was found to drive liver metastasis independent from steatosis *via* Y5R-mediated cAMP inhibition, ERK activation and CHK2 inactivation. While ERK-activation and cAMP inhibition mediated by Y5R had been confirmed recently by the inventors to drive hepatocellular carcinoma progression (Dietrich *et al.,* 2020), the existence of a potential NPY-Y5R-ERK/cAMP axis in liver metastasis had been unknown. Also, inactivation of the tumor suppressor CHK2 mediated by NPY-Y5R signaling had not been described before. CHK2 represents a potent tumor suppressor affecting metastasis in diverse types of cancer including melanoma, colorectal, pancreatic and prostate cancer (Pan *et al.,* 2019).

The inventors' studies show that liver metastasis is strongly reduced in NPY knockout mice, which is independent from steatosis. NPY is expressed by hepatocytes in close proximity of metastatic cancer cells and induced by TGF-β in the peri-metastatic microenvironment. In contrast to niche-associated upregulation of NPY, systemic NPY-levels are not altered in metastatic patients and mice. Metastatic cancer cells express NPY-receptors, and specifically NPY-5 receptor (Y5R) was found to regulate cAMP- and ERK-signaling after NPY binding, thereby inducing chemotactic migration and invasion. Moreover, Y5R-mediated dephosphorylation of checkpoint kinase 2 (CHK2) was identified to promote clonogenicity in metastatic cancer cells. In line with these findings, strong Y5R expression is associated with a metastatic phenotype in several types of cancers. *In vivo,* small molecule-mediated inhibition of Y5R as well as therapeutic RNA interference targeting NPY significantly reduced liver metastasis, as demonstrated in the examples herein.

Next to targeting the responsible receptor Y5R on metastatic cancer cells, RNAi appears as a particularly elegant strategy to target hepatocytes in the (pre-)metastatic liver niche, thereby reducing the chemotactic ligand NPY and counteracting potential resistance mechanisms when targeting Y5R (which might be induced by compensatory upregulation of NPY or by further NPY receptors). In pre-clinical mouse experiments, the inventors provided an LNP-mediated drug delivery *via* i.p. injection inducing stable gene knockdown in the liver. RNAi mediated targeting of NPY did not reveal liver-associated or systemic side effects, and both systemic levels of the metabolic regulator NPY as well as its expression in other tissues was unaffected.

Taken together, these findings reveal a novel cross-talk between hepatocytes and cancer cells in the metastatic liver niche, and show that inhibiting this cross-talk, for example by inhibition of Y5R or NPY, or by RNA interference targeting niche-associated NPY, is useful as a therapeutic approach to control liver metastasis.

Accordingly, the present invention relates to an inhibitor (herein referred to as 'NPY/Y5R inhibitor') of NPY-5 receptor activation by NPY for use in the treatment of liver metastasis in a subject.

The present invention also relates to a method of treating liver metastasis in a subject, wherein the method comprises administering to the subject an NPY/Y5R inhibitor.

The NPY/Y5R inhibitor used according to the present invention is selected from:
(i) one or more than one short interference RNA (siRNA) against NPY,
(ii) one or more than one Y5R inhibitor,
(iii) one or more than one anti-NPY antibody, and
(iv) one or more than one anti-Y5R antibody.

### DETAILED DESCRIPTION OF THE INVENTION

### Definitions

Unless otherwise defined herein, the singular article 'a' or 'an' means 'one or more than one', i.e. is not meant as a limitation to 'one'.

Unless otherwise defined herein, 'at least one' means 'one or more than one'

The term 'cancer' as used herein refers to malignant diseases characterized by the abnormal cell growth with the potential to spread and/or invade to other parts of the body. Said spreading/invasion of cancer cells from the primary cancer to a different site within the body is generally known as metastasis. In the context of the present invention, 'metastasis' in particular means metastasis to the liver ('liver metastasis'). Metastases are often found at the same time as the primary cancer, or later. In some subjects with metastases, a primary cancer is found only later or not at all, for example, because the (too small) size and/or the (unusual) location of the primary cancer prevented its diagnosis, or because the primary cancer regressed leaving behind its metastasis.

Cancers include solid tumors as well as non-solid cancers (e.g. leukemia). Particular non-limiting examples of primary cancers are biliary tract cancer, brain cancer, breast cancer, cervical cancer, choriocarcinoma, colorectal cancer (e.g., colon cancer), endometrial cancer, esophageal cancer, gastric cancer, intraepithelial neoplasm, leukemia, lymphoma, liver cancer, lung cancer, orolaryngeal cancer, malignant melanoma, myeloma, neuroblastoma, oral cancer, ovarian cancer, pancreatic cancer, prostate cancer, rectal cancer, sarcoma, skin cancer, stomach cancer, testicular cancer, thyroid cancer and renal cancer. The primary cancer can be different from primary liver cancer. In preferred embodiments, the primary cancer is selected from stomach cancer, colorectal cancer (e.g., colon cancer), breast cancer, pancreatic cancer, lung cancer, and malignant melanoma.

The terms 'treatment' and 'treating' as used herein with regard to liver metastasis refer to an intervention to prevent, reduce, and/or slow progression of liver metastasis in the treated subject by administering an NPY/Y5R inhibitor described herein to the subject. A treatment of liver metastasis as described herein can thus be a prophylactic treatment, i.e., a treatment of the subject to protect the subject against, or prevent the subject from suffering from, liver metastases. The treatment of liver metastasis can be the prevention or reduction of liver metastases in a subject who has or had a primary cancer, wherein said prevention or reduction can - at least in part - be achieved by one or more of the following activities of the NPY/Y5R inhibitor in the subject:
(a) inhibiting the migration and/or invasion of cancer cells from the primary cancer into the liver of the subject;
(b) inhibiting the growth of metastatic cancer cells of the primary cancer in the liver of the subject;
(c) reducing the colony formation capacity (clonogenicity) of metastatic cancer cells of the primary cancer in the liver of the subject.

Liver metastasis in a subject, or prevention or reduction of liver metastasis, can be determined, for example, by monitoring the number and/or the size of liver metastases over time using known techniques, e.g. MRT. Known tumor markers can be used to detect liver metastases and distinguish them from other tissues such as healthy liver tissues and primary liver cancer (primary liver tumors).

The NPY/Y5R inhibitor described herein can be administered to the subject (i) prior to treatment of the primary cancer (particularly as neoadjuvant treatment), (ii) during treatment of the primary cancer (particularly as peri-operative treatment), or (iii) after treatment of the primary cancer, or (iv) at two or all three of (i)-(iii). Administration of the NPY/Y5R inhibitor after treatment of the primary cancer can be as an adjuvant treatment or as a palliative treatment (e.g., years after removal of the primary cancer). For example, the NPY/Y5R inhibitor can be used in a combinatory treatment for synchronous or metachronous liver metastatic cancer.

The NPY/Y5R inhibitor can be used as a monotherapy, or can be combined with first- and/or later-line cancer treatments, for example with one or more of chemotherapy, immunotherapy, radiation and surgical cancer removal.

Unless specified otherwise herein, the terms 'subject' and 'patient' are used herein synonymously and refer to a human or a non-human animal, for example a companion animal. Non-limiting examples of subjects or patients which can be treated with an NPY/Y5R inhibitor as described herein are mammals such as humans, cats, dogs, and horses. Preferably, the subject or patient is a human. Typically, the subject is suspected or known to have a primary cancer or to have had a primary cancer in the past.

The inventors' studies demonstrated that treatment with an NPY/Y5R inhibitor as described herein can strongly reduce liver metastasis independent from steatosis. The subject treated with an NPY/Y5R inhibitor as described herein can therefore be a subject that has steatosis or a subject that does not have steatosis.

The NPY/Y5R inhibitor described herein is expediently administered in an effective amount. The term 'effective amount' as used herein in the context of a medical use or method of treating liver metastasis refers to the amount of NPY/Y5R inhibitor, or composition thereof, that is capable of eliciting the beneficial biological or medical response in a subject that is sought by the treating individual, i.e. medical doctor or other clinician, in particular a prophylactic or inhibitory effect against the formation of liver metastases, a reduction of the number of liver metastases and/or a reduction of the size of liver metastases.

For a treatment as described herein, the NPY/Y5R inhibitor can be administered in the form of a pharmaceutical composition comprising the NPY/Y5R inhibitor, a pharmaceutically acceptable carrier and, optionally, further excipients. The pharmaceutical composition can be formulated in any way that is compatible with its therapeutic application, including intended route of administration, delivery format and desired dosage.

Suitable administration routes for the NPY/Y5R inhibitor, or pharmaceutical composition thereof, include parenteral administration, for examples by injection. A preferred route of administration is subcutaneous administration. The optimal dose of the NPY/Y5R inhibitor administered will, of course, depend on the subject to be treated, particularly on the subject's weight, the subject's age, the manner of administration, and the judgement of the prescribing physician.

The term 'pharmaceutically acceptable' is used herein to refer to those compounds or compositions which are within the full range of medical judgment suitable for being administered to a human or non-human animal by the chosen route of administration, and thereby meet reasonable benefit / risk ratios without undue toxicity, irritation or other adverse events.

Neuropeptide Y (NPY), NPY-5 receptor (Y5R), and their encoding genes are known in the art (Larhammar *et al.,* 2004). For example, sequence information for the 36-amino acid human NPY, its precursor and encoding gene is found under NCBI accession no. P69101, Uniprot entry P01303 and NCBI gene ID 4852. NPY is a highly conserved protein, and known to act as a neuropeptide and tissue hormone expressed in the brain and sympathetic neurons of the peripheral nervous system. As described in the examples section herein, strong NPY expression has been found in peri metastatic liver tissues, especially in hepatocytes which are the major target of siRNA therapies. Sequence information for human Y5R is found under Uniprot entry Q15761 and NCBI gene ID 4889. As described in the examples section herein, strong Y5R expression has been found in cancer cells, in particular in metastatic cancer cells in the liver. An NPY/Y5R inhibitor as described herein in particular inhibits the activation of Y5R by NPY, and thereby the downstream signaling of NPY-Y5R crosstalk. Said downstream signaling is known to include activation of Gᵢ proteins resulting in potent antagonism of cAMP signaling (Dietrich *et al.,* 2020). An inhibitory effect on NPY-induced Y5R activation can thus, for instance, be measured by bioluminescence resonance energy transfer (BRET) analysis of forskolin-induced cAMP activation applying cancer cells *in vitro*; see cAMP BRET assay in the examples section herein. NPY-Y5R-induced ERK-induction in (samples of protein lysates prepared from) cell lines (for *in vitro* proof) or in peri-metastatic liver tissues (for *in vivo* proof), especially in hepatocytes, can be measured qualitatively and quantitatively by Western blot analysis, e.g. using an anti-phospho-ERK antibody; see examples section herein. Other measurable effects of (inhibition of) Y5R activation include (inhibition of) metastatic cancer cell migration and invasion as well as (inhibition of) time-dependent dephosphorylation of checkpoint kinase 2 (phospho-CHK2) at Thr 68, as described in the examples herein, which can also be measured qualitatively and quantitatively by Western blot analysis, e.g. using samples of protein lysates prepared from cell lines (for *in vitro* proof) or metastatic liver tissues (for *in vivo* proof), especially hepatocytes, and using an anti-phospo-Chk2 antibody (see examples section herein).

### SiRNA

The terms 'siRNA' and 'si-RNA" used herein are synonymous abbreviations of 'small interfering RNA' and refer to a double stranded RNA molecule which is capable of RNA interference (RNAi) of a target gene, particularly in a target-specific manner. Generally, it is believed that, after entering a target cell, the siRNA is introduced into the RNA-induced silencing complex (RISC), one strand of the siRNA ('sense' or 'passenger' strand) is removed, and the remaining strand ('antisense' or 'guide' strand) directed RISC towards sequence-specific binding to the target gene messenger RNA (mRNA). Said binding to the target gene mRNA results in cleavage and degradation of the mRNA, thus preventing synthesis of NPY protein (translation). See, e.g., Friedrich et al. (BioDrugs, 2022, 36, 549-571). The siRNA described herein is in particular synthetic siRNA.

The term 'siRNA against NPY' as used herein refers to an siRNA capable of RNAi of a gene encoding NPY, expediently the NPY gene of the subject to be treated. Administration of siRNA against NPY thus inhibits expression of NPY by RNAi, in particular NPY expression in hepatocytes.

RNAi therapeutics have already been approved and represent breakthrough discoveries in medicine (Strnad *et al.,* 2022; Balwani *et al.,* 2020). From 2018 to 2023, five siRNA drugs (Vutrisiran, Patisiran, Givosiran, Lumasiran, Inclisiran) were approved for the treatment of patients - all of them specifically target gene expression in hepatocytes (Adams *et al.,* 2018; Ahn *et al.,* 2023). The approved and known "in-pipeline" RNAi therapeutics are designed to treat hereditary disorders and metabolic (liver) diseases including amyloidosis, hypercholesteremia, alpha-1-anti-trypsine deficiency, NASH and diabetes (Strnad *et al.,* 2022; Ahn *et al.,* 2023). The concept of RNAi for treating cancer has not been evaluated in clinical phase II/III studies yet, mostly because siRNAs specifically target hepatocytes. This specific targeting, however, provides a particular advantage for the herein-described use of siRNAs against NPY in the treatment of liver metastasis by targeting NPY-expression of hepatocytes.

Design principles, advantageous chemical modifications, preparation methods and delivery for therapeutic siRNAs, which are applicable to the siRNAs against NPY used in the context of the present invention, are well known in the art. See, for, example, reviews by Hu et al. (Signal Transduction and Targeted Therapy, 2020, 5, 101) and Friedrich et al. (BioDrugs, 2022, 36, 549-571). Detailed information on design and modifications of siRNA is also found in Shmushkovich *et al.* (2018).

It is desirable to design siRNA such that any risk of immunostimulation is minimized. To this end, the siRNA should not contain immunostimulatory motifs, such as UGUGU. This and other putative immunostimulatory motif which should be avoided to minimize unwanted immune activation are described in Judge *et al.,* 2005.

Common features of known effective siRNAs (Patisiran, Givosiran, Inclisiran, Lumasiran, Vutrisiran, Fitusiran, Revusiran, Tivanisiran, Teprasiran and Cemdisiran) include an U at position 1 (associated with best silencing); no C at position 7 (because C at this position is associated with poor silencing and higher off-target levels); an A at position 10 (known to improve on-target silencing); no G at position 14; and a C at position 19 (associated with good silencing).

Each strand of an siRNA is typically 15-30 nucleotides in length. SiRNAs against NPY used in the context of the present invention are preferably 19-29 nucleotides in length, more preferably 19-25 nucleotides in length, and even move preferably 19-24 nucleotides in length. Further preferred properties of siRNAs include a GC content (of the entire siRNA or, preferably, of the antisense strand of the siRNA) of 30-60%, particularly 30-43%; asymmetry of the siRNA strands (i.e., the sense strand and the antisense strand of the siRNA have different lengths, in particular the sense strand is shorter than the antisense strand); a lack of palindromes, CCC and GGG sequences, and internal repeats; a lack of putative immunostimulatory motifs (e.g., as described in Judge *et al.,* 2005), such as UGUGU; a lack of single nucleotide polymorphisms in the target region (for parallel targeting of all SNP variants); a delta-delta-G of >0; an U at position 1; no G at position 14, particularly an A, an U or a C at position 14; no C at position 7, particularly an U, a G or an A at position 7, more particularly an A at position 7; a C at position 19; an A at position 10. Accordingly, siRNAs against NPY used in the context of the present invention are preferably characterized by one, two or all of the afore-mentioned preferred properties.

Exemplary siRNAs against human NPY include siRNAs having an antisense strand which comprises or consists of the nucleotide sequence set forth in any of SEQ ID NOs: 1-232 and SEQ ID NOs:263-292. Exemplary siRNAs against human NPY also include siRNAs having an antisense strand which comprises or consists of the nucleotide sequence set forth in any of SEQ ID NOs: 1-232, except that one or both of the last two nucleotides (nucleotides 20 and 21) of this sequence, independently, is missing or substituted by a different nucleotide.

Exemplary siRNAs against murine NPY are siRNAs having an antisense strand which comprises or consists of the nucleotide sequence set forth in any of SEQ ID NOs:233-262.

Additionally, the properties of siRNAs, including those of the exemplary siRNAs described herein, can be improved by chemical modifications of siRNA nucleotides. For example, such modifications can suppress immunostimulatory siRNA-driven activation of the innate immune response of the treated subject, improve chemical stability and efficacy, and/or decrease off-target-induced toxicity. SiRNAs against NPY used in the context of the present invention are therefore preferably characterized by one or more of such chemical modifications. For example, at least 50%, at least 60%, at least 70%, at least 80%, at least 90%, and in particular at least 100% of the nucleotides of an siRNA against NPY used in the context of the present invention are chemically modified as described herein.

According to the chemical structure of basic building blocks of natural RNA, said chemical modifications can be categorized as modifications in the ribose moiety ('ribose modification'), the phosphate backbone ('phosphonate modification'), and the nucleobases ('base modification'). The phosphonate modification may be one or more than one of methylphosphonate (MP), phosphorodithioate (PS2), methoxypropylphosphonate (MPO), 5'-phosphorothioate (5'-PS), (S)-5'-C-methyl with phosphate, peptide nucleic acid (PNA), phosphorothioate (PS) Rp isomer, phosphorothioate (PS) Sp isomer, 5'-(*E*)-vinylphosphonate (5'-(*E*)-VP), and 5'-methyl phosphonate (5'-MP). The ribose modification may be one or more than one of 2'-O-methyl (2'OMe), 2'-deoxy-2'-fluoro (2'F), 2'-methyl-4-pyridine (2'-O-CH2Py(4)), PMO, 2'-O-methoxyethyl (2'-O-MOE), tricyclo-DNA (tcDNA), Locked Nucleic Acid (LNA), 2'-arabino-fluoro (2'-Ara-F), 2'-O-benzyl, Glycol Nucleic Acid (GNA), Unlocked Nucleic Acid (UNA), and (S)-cEt-BNA. The ribose modification may be one or more than one of pseudouridine (Ψ), 2'-thiouridine (s2U), N6'-methyladenosine (m6A), N-ethylpiperidine 7'-EAA triazole modified adenine, N-ethylpiperidine 6'-triazole modified adenine, 6'-phenylpyrrolocytosine (PhpC), 5'-methylcytosine (m5C), 5'-fluoro-2'-deoxyuridine, 2',4'-difluorotoluyl ribonucleoside (rF), and 5'-nitroindole.

Known strategies for efficient delivery of siRNA to hepatocytes, which can be applied to the siRNAs against NPY used in the context of the present invention, include conjugation of the siRNA with N-acetyl galactosamine (GalNAc), and siRNA delivery in the form of siRNA-loaded lipid nanoparticles (LNPs). See, for, example, reviews by Hu et al. (Signal Transduction and Targeted Therapy, 2020, 5, 101) and Friedrich et al. (BioDrugs, 2022, 36, 549-571).

Delivery of the siRNAs in the form of GalNAc-siRNA conjugates is particularly preferred. GalNAc is a ligand of the asialoglycoprotein receptor (ASGPR), which is an endocytic receptor that is highly expressed on hepatocytes, and barely expressed by other cells. For example, in GalNAc-siRNA conjugates, trivalent or tetravalent GalNAc moieties are coupled to the 5'-end or 3'-end of the siRNA sense strand *via* a linker. GalNAc-siRNA conjugates are preferably administered subcutaneously.

Several classes of LNPs are known in the art and can be used for the delivery of siRNAs in the context of the present invention, including ionizable LNPs, cationic LNPs and neutral LNPs (Akinc *et al.,* 2019; Akinc *et al.,* 2010; de Fougerolles, 2008; Jayaraman *et al.,* 2012; Maestro *et al.,* 2021; Witzigmann *et al.,* 2020; Woitok *et al.,* 2020). Cationic LNPs and in particular ionizable LNPs are thereby particularly advantageous for siRNA delivery to hepatocytes. Examples of lipids which can be useful for the formation of LNPs for delivery of siRNAs in the context of the present invention include, but are not limited to, DLin-MC3-DMA ((6Z,9Z,28Z,31Z)-heptatriaconta-6,9,28,31-tetraen-19-yl 4-(dimethylamino)butanoate, CAS no. 1224606-06-7), e.g. in combination with cholesterol; DSPC ((2*R*)-2,3-bis(octadecanoyloxy)propyl 2-(trimethylazaniumyl)ethyl phosphate, CAS no. 816-94-4 (R enantiomer), CAS no. 4539-70-2 (racemate); PEG-DMG (1,2-dimyristoyl-rac-glycero-3-methylpolyoxyethylene; DLin-DMA (N,N-dimethyl-2,3-*bis*[(9Z,12Z)-9,12-octadecadien-1-yloxy]-1-propanamine, CAS no. 871258-12-7); and L-319 (9-[4-(dimethylamino)-1-oxobutoxy]-heptadecanedioic acid, 1,17-di-(2Z)-2-nonen-1-yl ester, CAS no: 1351586-50-9).

A NPY/Y5R inhibitor used in the context of the present invention can comprise one siRNA against NPY (this includes more than one identical siRNA molecules), or a combination of two or more different siRNAs against NPY, for example, 2, 3, 4, 5, 6, 7, 8, 9 or 10 different siRNAs against NPY.

### Y5R inhibitors which are small molecules

The term 'small molecule' as used herein refers to a compound having a molecular weight of not more than 1 kDa, for example from 0.1 kDa to 1 kDa.

Y5R inhibitors (Balasubramaniam *et al.,* 1997; Li *et al.,* 2015) which are useful in the context of the present invention include the following small molecules known to inhibit Y5R, for example:
*N*-[[*tran*s-4-[[(4-amino-2-quinazolinyl)amino]methyl]cyclohexyl]methyl]-1-naphthalenesulfonamide (CGP71683or a pharmaceutically acceptable salt thereof, for example CGP71683 hydrochloride (CAS no. 192322-50-2);
*trans*-N-[1-(2-fluorophenyl)-1H-pyrazol-3-yl]-1'-oxo-spiro[cyclohexane-1,3'(1'H)-furo[3,4-c]pyridine]-4-carboxamide (MK-0557; CAS no. 328232-95-7);
*N-*[4-methyl-9-(1-methylethyl)-9*H*-carbazol-3-yl]-4-morpholinecarboxamide (5RA972; CAS no. 439861-56-0);
*trans*-4-(tert-butylsulfonylamino)-N-[5-(trifluoromethyl)pyridin-2-yl]cyclohexane-1-carboxamide (S-2367, Velneperit; CAS no. 342577-38-2).

Whether or not a compound is an Y5R inhibitor can be determined as described herein, in particular: An inhibitory effect on NPY-induced Y5R activation can, for instance, be measured by bioluminescence resonance energy transfer (BRET) analysis of forskolin-induced cAMP activation applying cancer cells *in vitro*; see cAMP BRET assay in the examples section herein. NPY-Y5R-induced ERK-induction in (samples of protein lysates prepared from) cell lines (for *in vitro* proof) or in peri-metastatic liver tissues (for *in vivo* proof), especially in hepatocytes, can be measured qualitatively and quantitatively by Western blot analysis, e.g. using an anti-phospho-ERK antibody; see examples section herein. Other measurable effects of (inhibition of) Y5R activation include (inhibition of) metastatic cancer cell migration and invasion as well as (inhibition of) time-dependent dephosphorylation of checkpoint kinase 2 (phospho-CHK2) at Thr 68, as described in the examples herein, which can also be measured qualitatively and quantitatively by Western blot analysis, e.g. using samples of protein lysates prepared from cell lines (for *in vitro* proof) or metastatic liver tissues (for *in vivo* proof), especially hepatocytes, and using an anti-phospo-Chk2 antibody (see examples section herein).

### Antibodies against NPY or Y5R

Unless stated otherwise, the term 'antibody' as used herein refers to a (whole) immunoglobulin or an antigen-binding immunoglobulin fragment. Immunoglobulins are well known in the art and include antibodies such as, for example, IgA, IgD, IgE, IgG, and IgM. Antigen-binding immunoglobulins fragments are likewise well known in the art and include antigen-binding antibody fragments such as, for example, Fab, Fab', F(ab')₂, single domain antibodies (such as the variable domain of camelid heavy chain antibodies, VHH), and single chain Fv (scFv).

The term 'anti-NPY antibody' as used herein refers in particular to an antibody which is capable of specifically binding to NPY and, when bound to NPY, inhibit the activation of Y5R by NPY.

The term 'anti-Y5R antibody' as used herein refers in particular to an antibody which is capable of specifically binding to Y5R and, when bound to Y5R, inhibit the activation of Y5R by NPY.

Techniques for generating antibodies against a known target antigen are well known in the art, and can be applied to generate antibodies against NPY or Y5R. Techniques for generating the antigen material for such purpose are likewise well known in the art, and can be applied for generating antigen material for the identification of anti-NPY antibodies or anti-Y5R antibodies. Such techniques include, for example, recombinant expression and purification techniques for generating a known protein (such as NPY or Y5R) or an antigenic portion thereof, and techniques for generating cells presenting a target antigen which is a receptor protein (such as Y5R) at their surface.

Whether or not an anti-NPY antibody or an anti-Y5R antibody (by binding NPY or Y5R, respectively) inhibits the activation of Y5R by NPY can be determined as described herein, in particular: An inhibitory effect on NPY-induced Y5R activation can, for instance, be measured by bioluminescence resonance energy transfer (BRET) analysis of forskolin-induced cAMP activation applying cancer cells *in vitro*; see cAMP BRET assay in the examples section herein. NPY-Y5R-induced ERK-induction in (samples of protein lysates prepared from) cell lines (for *in vitro* proof) or in peri-metastatic liver tissues (for *in vivo* proof), especially in hepatocytes, can be measured qualitatively and quantitatively by Western blot analysis, e.g. using an anti-phospho-ERK antibody; see examples section herein. Other measurable effects of (inhibition of) Y5R activation include metastatic cancer cell migration and invasion as well as (inhibition of) time-dependent dephosphorylation of checkpoint kinase 2 (phospho-CHK2) at Thr 68, as described in the examples herein, which can also be measured qualitatively and quantitatively by Western blot analysis, e.g. using samples of protein lysates prepared from cell lines (for *in vitro* proof) or metastatic liver tissues (for *in vivo* proof), especially hepatocytes, and using an anti-phospo-Chk2 antibody (see examples section herein).

### DESCRIPTION OF THE FIGURES

Figure 1 A-B. Boyden chamber analysis and representative images (10-fold original magnification) of chemotaxis of human melanoma cells (SkMel28) towards recombinant NPY (lower chamber) with or without application of Y5R specific inhibitor CGP71683 (Y5Ri) (upper chamber) compared to migration towards serum free medium (n = 9). Arrowheads indicate migrated cells.
Figure 2. Representative images of colony number and colony size of human melanoma cells (SkMel28) in clonogenicity assays with application of increasing concentrations of Y5R inhibitor (Y5Ri) (n = 3).
Figure 3. Schematic illustration of the experimental procedure: splenic injection of murine melanoma cells (B16) in C57BL/6 mice on day 0; intraperitoneal injection of Y5R inhibitor CGP71683 (Y5Ri) (15 mg/kg body weight per injection) or solvent only (DMSO) as control on indicated days; end of experiment eight days after cell implantation.
Figure 4. Explanted metastases bearing spleens and livers of DMSO (control) and Y5Ri treated mice after splenic injection of B16 melanoma cells.
Figure 5. Macroscopic classification of livers from DMSO (n = 11) and Y5Ri treated mice (n = 9) into very low / low versus moderate / high liver metastasis.
Figure 6. A. CHK2 level in NPY^{-/-} mice compared to control WT mice measured by IHC analysis. B. CHK2 level in Y5Ri-treated mice compared to control DMSO-treated mice measured by IHC analysis.
Figure 7. Schematic illustration of the experimental procedure: pre-treatment of C57BL/6 mice with lipid nanoparticles (LNP) bearing control siRNA pool or siNPY pool (pool of siRNAs targeting murine NPY) one day before surgery (day -1); splenic injection of murine melanoma cells (B16) on day 0; intraperitoneal injection of LNPs with control siRNA pool or siNPY pool as indicated; end of experiment six days after cell implantation.
Figure 8. A. Quantification of NPY mRNA expression levels in liver tissues from mice treated with control LNPs ("Control", LNPs bearing control siRNA pool, n = 11) and from mice treated with LNPs bearing siRNA pool targeting murine NPY ("LNP-siNPY", n = 11). B. Quantification of NPY protein levels in liver tissues from mice treated with control LNPs (n = 11) and LNP-siNPY treated mice (n = 11).
Figure 9 A-B. ELISA based protein expression analysis of absolute NPY levels in liver tissues and serum samples from LNP-siNPY treated mice (n = 10) compared to mice treated with control LNPs (LNPs bearing control siRNA pool, n = 11) after splenic melanoma cell injection.
Figure 10. Analysis of relative spleen (A) and liver (B) weight after splenic injection of B16 melanoma cells and i.p. treatment with LNP-siNPY (n = 11) compared to mice treated with control LNPs (LNPs bearing control siRNA pool, n = 11).
Figure 11. Representative images of livers from mice treated with control LNPs (LNPs bearing control siRNA pool, n = 11) and LNP-siNPY treated mice (n = 11).
Figure 12. A. Quantification of number of metastases in liver tissues from LNP-siNPY treated mice (n = 10) compared to mice treated with control LNPs (LNPs bearing control siRNA pool, n = 11). B. Quantification of metastases size in liver tissues from LNP-siNPY treated mice (n = 10) compared to mice treated with control LNPs (LNPs bearing control siRNA pool, n = 11).
Figure 13. Classification of livers from mice treated with control LNPs (LNPs bearing control siRNA pool, n = 11) and LNP-siNPY treated mice (n = 11) into very low / low versus moderate / high liver metastasis.
Figure 14. A. Quantification of pERK protein expression in metastatic liver tissue samples of LNP-siNPY treated mice (n = 6) compared to mice treated with control LNPs (LNPs bearing control siRNA pool, n = 6). B. Quantification of pCHK2 protein expression in metastatic liver tissue samples of LNP-siNPY treated mice (n = 6) compared to samples from control treatment (control siPool bearing LNPs) (n = 6).
Figure 15. NPY mRNA expression in primary human hepatocytes after transfection with a siRNA pool against human NPY ("siNPY") or a negative control siRNA pool ("siCtr") for 24 hours. (n=3 independent experiments; *: p<0.01).

### EXAMPLES

### A Experimental Procedures

### A.1 siRNA pools

The examples use the following siRNA pools:
- control siRNA pool of 30 non-targeting siRNAs having the antisense strand sequences of SEQ ID NOs: 321-350
- siRNA pool targeting murine NPY: pool of 30 siRNAs having the antisense strand sequences of SEQ ID NOs: 233-262
- siRNA pool targeting human NPY: pool of 30 siRNAs having the antisense strand sequences of SEQ ID NOs: 263-292

### A.2 Lipid nanoparticle mediated siRNA pool delivery

Lipid nanoparticles (LNP) were designed as described in detail by Evers *et al.,* 2022. LNPs are considered promising drug delivery vehicles for liver specific clinical siRNA delivery (Evers *et al.,* 2022). LNPs were prepared by microfluidic mixing using the NanoAssemblr Benchtop (Precision Nanosystems, Vancouver, Canada). An ethanolic phase containing lipids was mixed with an acidic aqueous phase (100 mM sodium acetate, pH 4.0) containing siRNA pools leading to the formation of LNPs. LNPs were produced at a flow rate ratio (aqueous:organic) of 3:1 and a total flow rate of 4.0 mL/min. Lipids were dissolved in 100% Ethanol (Merck, Darmstadt, Germany) at a total lipid concentration of 10 mM. The LNPs were composed of DLin-MC3-DMA ((6Z,9Z,28Z,31Z)-heptatriaconta-6,9,28,31-tetraen-19-yl 4-(dimethylamino)butanoate, CAS no. 1224606-06-7), Cholesterol (Sigma Aldrich, Saint Louis, MO, USA), DSPC ((2*R*)-2,3-bis(octadecanoyloxy)propyl 2-(trimethylazaniumyl)ethyl phosphate, CAS no. 816-94-4 (R enantiomer), CAS no. 4539-70-2 (racemate); Lipoid, Ludwigshafen am Rhein, Germany) and PEG-DMG (1,2-dimyristoyl-rac-glycero-3-methylpolyoxyethylene; NOF Corporation, Tokyo, JP) at a molar percentage of 50/38.5/10/1.5, respectively. siRNA pools were encapsulated at an N/P ratio (ionizable lipid/RNA) of 6:1. Immediately after production, LNPs were dialyzed against an excess of phosphate buffered saline using Slide-a-Lyzer^{™} dialysis cassettes G2 with a membrane cutoff of 20 kDa for 16-24 h. After dialysis, LNPs were sterilized using 0.45 µm PVDF membrane filters. Purified LNP was kept at 4°C and used within 40 days after production. LNPs containing a specifically designed si-RNA pool targeting murine NPY were applied in preliminary experiments comparing tail vein vs. subcutaneous vs. i.p. (intraperitoneal) injection. I.p. injection was sufficient to significantly reduce NPY expression in the liver, while no effects were observed in other tissues or in the blood.

### A.3 Animal models

Animals were obtained from Charles River Laboratories and housed under specific pathogen-free and controlled conditions (22°C, 55% humidity, 12 h day/night rhythm, free access to food and water). Melanoma tissues from a xenograft tumor model were obtained and described in previous studies (Dietrich *et al.,* 2018). A murine spleen-liver metastasis model was used as previously described (Koch *et al.,* 2015). Briefly, this model was performed by intrasplenic injection of B16 melanoma or CT26 colorectal carcinoma cells, respectively (1.5 × 10⁵ in 5 µl serum-free medium).

The following conditions were applied for pharmacologic inhibition of the Y5R using the specific small-molecule Y5R inhibitor CGP71683 ("Y5Ri"): Starting on the first day after cell implantation, mice of the Y5Ri group received a single dose (15 mg/kg bodyweight) every 24 h by intraperitoneal (i.p.) injection, while mice of the control group received the vehicle (ethanol (50%), DMSO (50%)).

For *in vivo* analysis of the effects of NPY depletion on melanoma-derived liver metastasis, the spleen-liver metastasis model was conducted using 129S-Npytm1Rpa/J mice after crossing with C57BL/6 mice to archive immunotolerance to the B16 cell line (BL/6 background). The experiment was terminated on day seven after cell implantation.

Applying a further model of metastasis, RNA interference-mediated, therapeutic NPY knockdown was established using i.p. injection of a complex consisting of lipid nanoparticles and a siRNA pool against murine NPY ("LNP-siNPY"). Mice of the control group received a complex of lipid nanoparticles and a control siRNA pool. I.p. injections were conducted on the day before and on the first, third and fifth day after cell implantation. The experiment was terminated six days after cell implantation.

In a further series of experiments, induction of mild steatosis prior to metastasis formation was applied. Mice were fed a high-fat Western-type diet (38% Fat, 15% Lard, 15% Tallow, 4% C16:0, 4% C18:0) for 6 weeks. Subsequently, the spleen-liver metastasis model was performed as described herein.

In all experiments, mice were killed by heart puncture under deep ketamine/xylazine-induced anesthesia. Blood samples were collected and further processed to obtain plasma samples. Non-tumorous and tumorous spleen and liver tissues as well as lung and gut tissue samples were immediately snap-frozen and stored at -80°C or were formalin-fixed for immunohistochemical analysis. All experimental protocols were approved by the Committee for Animal Health and Care of the local government and conformed to international guidelines on the ethical use of animals.

### A.4 Human patient tissues

Patient tissues were collected after written informed consent was obtained through the Human Tissue and Cell Research Foundation (HTCR). Snap-frozen samples were immediately stored at -80°C. A tissue microarray consisting of tissues from nevi, primary melanoma and melanoma metastases was described previously (Koch *et al.,* 2015). Primary melanoma tissues from patients with or without liver metastases were obtained from the Biobank of the Department of Dermatology, Universitätsklinikum Erlangen. Tissues samples from primary tumors of melanoma, breast cancer and pancreatic cancer patients, as well as tissues from liver metastases of melanoma, breast cancer, pancreatic cancer, and colorectal cancer patients were obtained from obtained from the Department of Surgery within the LiquImm study. Clinicopathological features of the mentioned tissues are listed in Tables 1-3.

**Table 1. Patient characteristics. BC: Breast cancer. CRC: Colorectal cancer. IR: immunoreactivity (score). MET: Metastatic tissue. MM: Malignant melanoma. Nd: not determined / missing. Other: Further types of cancer (i.e. gastric cancer (n=1), renal cancer (n=3), cholangiocellular carcinoma, fibrosarcoma (n=3), fibrosarcoma (n=1)). PC: Pancreatic cancer. Peri-tumor: Tumor-surrounding liver tissue.**

| **Clinico-pathological feature** | **Categorization** | ***n* (%)** |
|---|---|---|
| Age at diagnosis | <65 years | 19 (50.0) |
| | ≥65 years | 13 (34.2) |
| | Nd | 6 (15.8) |
| | total | 38 (100) |
| Gender | Female | 19 (50.0) |
| | Male | 19 (50.0) |
| | total | 38 (100) |
| Tumor type | CRC | 18 (47.4) |
| | MM | 8 (21.0) |
| | PC | 2 (5.3) |
| | BC | 2 (5.3) |
| | Other | 8 (21.0) |
| | Nd | 38 (100) |
| | total | |
| NPY IR (peri-tumor) | 0 (negative) | 0 (0.0) |
| | 1+ | 6 (15.8) |
| | 2+ | 11 (28.9) |
| | 3+ | 21 (55.3) |
| | Nd | 0 |
| NPY IR (MET) | 0 (negative) | 22 (57.9) |
| | 1+ | 12 (31.7) |
| | 2+ | 1 (2.6) |
| | 3+ | 1 (2.6) |
| | Nd | 2 (5.2) |
| Y5R IR (MET) | 0 (negative) | 11 (28.9) |
| | 1+ | 7 (18.4) |
| | 2+ | 6 (15.8) |
| | 3+ | 13 (34.3) |
| | Nd | 1 (2.6) |
| Y1R IR (MET) | 0 (negative) | 11 (28.9) |
| | 1+ | 13 (34.3) |
| | 2+ | 5 (13.2) |
| | 3+ | 0 (0.0) |
| | Nd | 9 (23.6) |
| phospho-ERK IR (MET) | 0 (negative) | 22 (57.9) |
| | 1+ | 4 (10.5) |
| | 2+ | 6 (15.8) |
| | 3+ | 4 (10.6) |
| | Nd | 2 (5.2) |
| Ki-67 IR (MET) | 0 (negative) | 0 (0.0) |
| | 1+ | 17 (44.8) |
| | 2+ | 11 (28.9) |
| | 3+ | 9 (23.7) |
| | Nd | 1 (2.6) |
| phospho-CHK2 IR (MET) | 0 (negative) | 19 (50.0) |
| | 1+ | 9 (23.7) |
| | 2+ | 6 (15.7) |
| | 3+ | 0 (0.0) |
| | Nd | 4 (10.6) |
| TGF-β IR (peri-tumor) | 0 (negative) | 0 (0.0) |
| | 1+ | 3 (7.9) |
| | 2+ | 14 (36.8) |
| | 3+ | 18 (47.4) |
| | Nd | 3 (7.9) |
| TGF-β IR (MET) | 0 (negative) | 20 (52.6) |
| | 1+ | 7 (18.5) |
| | 2+ | 4 (10.6) |
| | 3+ | 1 (2.6) |
| | Nd | 6 (15.7) |

**Table 2. Clinicopathological characteristics of melanoma patients (systemic NPY levels). Nd: not determined / missing**

| **Clinico-pathological feature** | **Categorization** | ***n* (%)** |
|---|---|---|
| Age at diagnosis | <65 years | 117 (51.5) |
| | ≥65 years | 110 (48.5) |
| | total | 227 (100.0) |
| Gender | Female | 116 (50.1) |
| | Male | 111 (48.9) |
| | total | 227 (100.0) |
| Melanoma type | Uveal | 177 (78.0) |
| | Cutan | 48 (21.1) |
| | Nd | 2 (0.9) |
| | total | 227 (100.0) |
| Site of metastasis | None | 111 (48.9) |
| | Non-liver | 20 (8.8) |
| | Liver | 39 (17.2) |
| | Non-liver and liver | 56 (24.7) |
| | Nd | 1 (0.4) |
| | total | 227 (100.0) |

**Table 3. Clinicopathological characteristics and immunoreactivity in primary tumor tissues (TMA). BC: Breast cancer. CRC: Colorectal carcinoma. IR: immunoreactivity (score). MM: Malignant melanoma. Nd: Not determined/missing. PC: Pancreatic cancer**

| **Clinico-pathological feature** | **Categorization** | ***n* (%)** |
|---|---|---|
| All cancers | MM | 252 (24.8) |
| (MM, CRC, PC, BC) | CRC | 351 (34.6) |
| | PC | 176 (17.4) |
| | BC | 235 (23.2) |
| | total | 1,014 (100.0) |
| Melanoma (MM) | Yes | 12 (4.8) |
| Liver METs | No | 240 (95.2) |
| | total | 252 (100.0) |
| Melanoma (MM) | Yes | 36 (14.3) |
| Liver MET "high risk" | No | 216 (85.7) |
| (Uveal and mucosal melanoma) | total | 252 (100.0) |
| Colorectal cancer (CRC) Liver METs | Yes | 64 (18.2) |
| | No | 287 (81.8) |
| | total | 351 (100.0) |
| Pancreatic cancer (PC) Liver METs | Yes | 13 (7.4) |
| | No | 163 (92.6) |
| | total | 176 (100.0) |
| Breast cancer (BC) | Yes | 3 (1.3) |
| Liver METs | No | 232 (98.7) |
| | total | 235 (100.0) |
| Melanoma (MM) | 0 (negative) | 91 (36.1) |
| Y5R IR | 1+ | 67 (26.6) |
| | 2+ | 52 (20.6) |
| | 3+ | 42 (16.7) |
| | total | 252 (100.0) |
| Colorectal cancer (CRC) | 0 (negative) | 64 (18.2) |
| Y5R IR | 1+ | 147 (41.9) |
| | 2+ | 114 (32.5) |
| | 3+ | 26 (7.4) |
| | total | 351 (100.0) |
| Pancreatic cancer (PC) | 0 (negative) | 84 (47.7) |
| Y5R IR | 1+ | 46 (26.1) |
| | 2+ | 30 (17.1) |
| | 3+ | 16 (9.1) |
| | total | 176 (100.0) |
| Breast cancer (BC) | 0 (negative) | 73 (31.1) |
| Y5R IR | 1+ | 55 (23.4) |
| | 2+ | 56 (23.8) |
| | 3+ | 51 (21.7) |
| | total | 235 (100.0) |
| Melanoma (MM) | 0 (negative) | 102 (40.5) |
| pERK IR | 1+ | 72 (28.6) |
| | 2+ | 39 (15.5) |
| | 3+ | 20 (7.9) |
| | Nd | 19 (7.5) |
| | total | 252 (100.0) |
| Colorectal cancer (CRC) | 0 (negative) | 209 (59.5) |
| pERK IR | 1+ | 62 (17.7) |
| | 2+ | 46 (13.1) |
| | 3+ | 13 (3.7) |
| | Nd | 21 (6.0) |
| | total | 351 (100.0) |
| Pancreatic cancer (PC) | 0 (negative) | 85 (48.3) |
| pERK IR | 1+ | 46 (26.1) |
| | 2+ | 30 (17.1) |
| | 3+ | 13 (7.4) |
| | Nd | 2 (1.1) |
| | total | 176 (100.0) |
| Breast cancer (BC) | 0 (negative) | 116 (49.4) |
| pERK IR | 1+ | 54 (23.0) |
| | 2+ | 42 (17.9) |
| | 3+ | 21 (8.9) |
| | Nd | 2 (0.8) |
| | total | 235 (100.0) |

### A.5 Organoid model

Murine APTAK organoids were used for *in vitro* and *in vivo* experiments. These APTAK tumor organoids are deficient in Apc, Tp53, Tgfbr2 and express constitutively activated Kras and activated Akt1. Organoids were generated and CRC models were conducted in a similar way as previously described (Heichler *et al.,* 2020). In an orthotopic mouse model for CRC and CRC (liver) metastasis, single cell suspensions from genetically engineered APTAK organoids were injected into the submucosa of wildtype mice.

### A.6 Statistical analysis

Results were expressed as mean ± SEM. In order to compare groups, 2-tailed Student's t-tests or 1-way ANOVAs were used, along with Dunnett's multiple comparison test when appropriate. Correlation analysis was conducted using Spearman's and Pearson's coefficients, respectively. To analyze tissue microarrays, Fisher's exact test and Qui-Square test, as well as Spearman correlation analysis, and uni- and multivariate analysis applying the SPSS ordinal regression procedure (Polytomous Universal Model [PLUM]; link function: logit) were used. *In silico* survival analysis was performed by computationally applying log-rank testing and hazard ratio estimates. A P value less than 0.05 was considered significant. Figures display the significance level as *P < 0.05, **P < 0.01, ***P < 0.001, and ****P < 0.0001. The number of experiments is depicted in the figure legends. Calculations were performed using Prism software (GraphPad Software Inc.) and SPSS (SPSS Statistics 23, IBM Corp.).

### A.7 Cell culture

Normal human epidermal melanocytes (NHEM), the human primary melanoma cell lines Mel Juso, Mel Ei, Mel Wei and metastatic human melanoma cell lines SkMel28 (ATCC CRL-1469^{™}), Mel Im, Mel Ju, SkMel3 (ATCC HTB-69^{™}) were described previously (Jacob *et al.,* 1998; Heimerl *et al.,* 2007). Primary human hepatocytes (PHH) were isolated and cultured as described elsewhere (Dietrich *et al.,* 2017; Lee *et al*., 2013). In addition, the human breast cancer cell line MCF-7 (ATCC HTB-22^{™}), the human pancreatic cancer cell line Panc-1 (ATCC CRL-1469^{™}) and the human colon cancer cell lines HT29 (ATCC HTB-38^{™}), Caco-2 (ATCC HTB-37^{™}) and LoVo (ATCC CCL-229^{™}) were used. The murine melanoma cell line B16 (ATCC CRL-6475^{™}) was used for *in vitro* and *in vivo* studies. Furthermore, the murine pancreatic cancer cell line KPC and the murine colon cancer cell lines MC38 and CT26 (ATCC CRL-2638^{™}) were used. Cell lines and characteristics are listed in Table 4.

**Table 4. Summary and additional information of human and murine cell lines. BC: Breast cancer. CRC: Colorectal cancer. Hetero: Heterozygous genotype. Homo: Homozygous genotype. MET: Metastatic melanoma cell line. MM: Malignant melanoma. Nd: not determined / missing. PC: Pancreatic cancer. PT: Primary tumor melanoma cell line. Peri-tumor: Tumor-surrounding liver tissue. IR: immunoreactivity (score).**

| **Name of cell line** | **Reference** | **Species** | **Tumor** | **Cell culture medium/conditions** | **BRAF mutation status** |
|---|---|---|---|---|---|
| **Mel Im** | Johnson et al., 1981 | human | MM (MET) | DMEM low, 10% FCS | V600E (homo) |
| **Mel Ju** | Johnson et al., 1981 | human | MM (MET) | DMEM low, 10% FCS | V600E |
| **Mel Ei** | Johnson et al., 1981 | human | MM (PT) | DMEM low, 10% FCS | V600E (homo) |
| **Mel Wei** | Johnson et al., 1981 | human | MM (PT) | DMEM low, 10% FCS | V600E |
| **Mel Juso** | Johnson et al., 1981 | human | MM (PT) | RPMI, 10% FCS | V600E (hetero) |
| **SkMel-3** | ATCC: HTB-69 | human | MM (MET, lymph node) | DMEM low, 10% FCS | V600E (hetero) |
| **SkMel-28** | ATCC: HTB-72 | human | MM (MET) | DMEM low, 10% FCS | V600E |
| **B16** | ATCC: CRL-6475 | murine | MM | DMEM high, 10% FCS | |
| **Caco-2** | ATCC: HTB-37 | human | CRC | DMEM high, 10% FCS | |
| **HT29** | ATCC: HTB-38 | human | CRC | DMEM low, 10% FCS | |
| **LoVo** | ATCC: CLL-229 | human | CRC | DMEM high, 10% FCS | |
| **CT26** | ATCC: CRL-2638 | murine | CRC | DMEM high, 10% FCS | |
| **MC38** | Corbett et al., 1975 | murine | CRC | DMEM high, 10% FCS | |
| **Panc-1** | ATCC: CRL-1469 | human | PC | RPMI, 10% FCS | |
| **KPC** | Krebs et al., 2017 | murine | PC | DMEM high, 10% FCS | |
| **MCF-7** | ATCC: HTB-22 | human | BC | DMEM high, 10% FCS | |

### A.8 Treatment with recombinant proteins, agonists, inhibitors, and antibodies

As described in the examples, figures and figure legends, recombinant proteins, small molecule agonists and antagonists were used in this study. TGF-β was purchased from R&D Systems (Minneapolis, USA). The activation of Y5R was conducted using the synthetic high-affinity Y5R-specific-agonist BWX46 ("Y5R-Ago") (CAS-No. 172997-92-1; Tocris, Wiesbaden, Germany) and recombinant neuropeptide Y ("NPY") (Merck, Darmstadt, Germany). To inhibit Y5R-signaling with high affinity and selectivity over other NPY-receptors, the selective high-affinity small molecule inhibitor CGP71683 ("Y5R-Inh") (CAS-No. 192322-50-2; Tocris) was used. For Y1R-inhibition, the specific small molecule inhibitor BIBO 3304 (Tocris) ("Y1R-Inh") was used. For Y2R-inhibition, the specific small molecule inhibitor BIIE 0246 (CAS-No. 246146-31-6, Tocris) ("Y2R-Inh") was used. Forskolin (Merck) was used to elevate intracellular cAMP levels (Dietrich *et al.,* 2020). Cinobufagin (MedChemExpress, New Jersey, USA) was applied for dose-dependent induction of phospho-CHK2 (Pan *et al.,* 2019). The MEK inhibitor U0126 (Merck) was used to specifically inhibit ERK activation.

### A.9 siRNA pool mediated gene knockdown

Briefly, for *in vitro* transfection, in each well (six-well plates), 2 × 10⁴ cells were seeded. Lipofectamine RNAimax transfection reagent (Life Technologies, Darmstadt, Germany) was applied as described (Dietrich *et al.,* 2017). In order to knock down the expression of Y5R, a functionally verified siRNA pool targeting murine NPY or a functionally verified siRNA pool targeting human NPY (siTOOLs Biotech GmbH, Planegg, Germany; see information in section A.1 herein) was used. The siRNA pools comprised 30 target-specific single siRNAs, allowing for highly efficient gene silencing as compared with the non-targeting control siRNA pool, while off-target effects of individual siRNAs can be reduced to a minimum (Hannus *et al.,* 2014). RNA and protein were isolated and functional assays were conducted 24 after transfection.

### A.10 Immunohistochemistry & Immunofluorescence

Immunohistochemistry (IHC) and immunofluorescence analyses were performed using tissue microarrays consisting of human cancer tissue samples and mouse paraffin-embedded tissue sections as described previously (Dietrich *et al.,* 2017; Dietrich *et al.,* 2020). The following primary antibodies were used: anti-alpha-SMA antibody (catalog no. ab5694, Abcam; 1 in 500 dilution), anti-CD3 antibody (catalog no. C7930, Sigma-Aldrich, Saint Louis, Missouri, USA; 1 in 1000 dilution), anti-CD31 antibody (catalog no. DIA-310, Dianova GmbH, Hamburg, Germany; 1 in 50 dilution), anti-CD45 antibody (catalog no. 14-0452, Affymetrix, Santa Clara, California, USA; 1 in 50 dilution), anti-CD4 antibody (catalog no. 14-9766, Affymetrix; 1 in 100 dilution), anti-CD8 antibody (catalog no. 14-0808, Affymetrix; 1 in 50 dilution), anti-cleaved-caspase antibody (Cell Signaling, Frankfurt am Main, Germany; 1 in 50 dilution), anti-phospho-ERK antibody (catalog no. #9101, Cell Signaling; 1 in 100 dilution), anti-Ki-67/MIB-1 antibody (catalog no. M7240, Dako GmbH, Hamburg, Germany; 1 in 50 dilution), anti-Y5R antibody (catalog no. ab133757, Abcam; 1 in 1,000 dilution), anti-NPY antibody (catalog no. ab6173, Abcam; 1 in 500 dilution), anti-NPY antibody (catalog no. HPA044572, Sigma-Aldrich; 1 in 200 dilution), anti-NPY antibody (catalog no. ab10341, Abcam; 1 in 1,000 dilution), anti-NPY antibody (catalog no. BML-NA1233, Enzo Life Sciences, Farmingdale, USA; 1 in 500 dilution), anti-TGFβ1 antibody (catalog no. DM1047, Acris Antibodies; Herford, Germany; 1 in 100 dilution), anti-phospho-Chk2 antibody (catalog no. #2661, Cell Signaling; 1 in 50 dilution). For each antibody, immunohistochemistry staining was evaluated semi-quantitatively and scores were calculated as described previously (Dietrich *et al.,* 2017; Dietrich *et al.,* 2020).

### A.11 Western blotting

As described in detail in previous studies (Dietrich *et al.,* 2017), Western blots and densitometric quantification were performed using the following primary antibodies: anti-β-actin antibody (catalog no. A1978, Sigma-Aldrich; 1 in 5,000 dilution), anti-phospho-ERK antibody (catalog no. #9101, Cell Signaling, Frankfurt am Main, Germany; 1 in 4,000 dilution), anti-ERK antibody (catalog no. #9102, Cell Signaling; 1 in 1,000 dilution), anti-phospho-AKT antibody (catalog no. #9271, Cell Signaling, Frankfurt am Main, Germany; 1 in 2000 dilution), anti-AKT (catalog no. #9272, Cell Signaling, Frankfurt am Main, Germany; 1 in 2000 dilution), anti-Y5R antibody (catalog no. ab133757, Abcam; 1 in 30,000 dilution), anti-phospho-Chk2 antibody (catalog no. #2661, Cell Signaling, Frankfurt am Main, Germany; 1 in 1000 dilution), anti-phospho-p53 antibody (catalog no. #9284, Cell Signaling, Frankfurt am Main, Germany; 1 in 1000 dilution).

### A.12 RNA isolation and reverse transcription

Using the E.Z.N.A. Micro Elute Total RNA Kit (Omega, Norcross, GA, USA), RNA was isolated as described elsewhere (Dietrich *et al.,* 2017). Using the SuperScript II Reverse Transcriptase Kit, cDNAs of total RNA fractions were generated (Invitrogen, Groningen, Netherlands).

### A.13 Analysis of mRNA-expression by quantitative RT-PCR

As described previously (Dietrich *et al.,* 2017; Dietrich *et al.,* 2020), quantitative RT-PCR was performed using the following primer pairs: 18S rRNA (5'-TCT GTG ATG CCC TTA GAT GTC C-3' and 5'-CCA TCC AAT CGG TAG TAG CG-3'; SEQ ID NOs:293 and 294), CyclinD1 (5'-GCC TGT GAT GCT GGG CAC TTC ATC TG-3' and 5'-TTT GGT TCG GCA GCT TGC TAG GTG AC-3'; SEQ ID NOs:295 and 296), Npy (murine) (5'-TGG CCA GAT ACT ACT CCG CT-3' and 5'-GCA GAC TGG TTT CAG GGG AT-3'; SEQ ID NOs:297 and 298), NPY (human) (5'-GCT AGG TAA CAA GCG ACT GGG-3' and 5'-TGG GCT GGA TCG TTT TCC AT-3'; SEQ ID NOs: 299 and 300), Npy1r (murine) (5'-CAC CTG CAA CCA CAA TCT GC-3' and 5'-GAC GTC TTG GAC ACA TCC GT-3'; SEQ ID NOs:301 and 302), NPY1R (human) (5'-AAT GCT TCC CCG AGG TG TG-3' and 5'-TGG GGA AGA TCC GCT GGT AT-3'; SEQ ID NOs:303 and 304), Npy2r (murine) (5'-GGA TCT GCA TCA GGC ACA CT-3' and 5'-CGC CTT AGG TAG CAA GCC AT-3'; SEQ ID NOs:305 and 306), NPY2R (human) (5'-CTC CCT CGC CAC CAA AAC TTC-3' and 5'-GTG AAG GTG GGA GGA TCA GA GA-3'; SEQ ID NOs:307 and 308), Npy5r (murine) (5'-GGT ACA GCA AGA AGA CGG CA-3' and 5'- TTC TCG TGA GGG AAC GCT TG-3'; SEQ ID NOs:309 and 310), NPY5R (human) (5'-AAT ACT GCT GCC ACT CGG AA-3' and 5'-GGC CAG ATT GCC TAT GAG GA-3'; SEQ ID NOs:311 and 312), TGF-β1 (human) (5'-GAG ATG GCA GGG ACT CTG ATA ACA CC-3' and 5'-AAA GTG CTA GGA TTA CAG GCG TGA GC-3'; SEQ ID NOs:313 and 314), Tgf-β1 (murine) (5'-AGG AGA CGG AAT ACA GG GC-3' and 5'-CCA CGT AGT AGA CGA TGG GC3'; SEQ ID NOs:315 and 316), gp70 (murine) (5'-AGC CCC TTG AGA CTA CGG GT-3' and 5'-CGT CAA TGG GAA CCT GTG CG-3'; SEQ ID NOs:317 and 318), aSMA (murine) (5'-CCA GCC ATC TTT CAT TGG GAT-3' and 5'-CCC CTG ACA GGA CGT TGT TA-3'; SEQ ID NOs:319 and 320).

### A.14 Clonogenicity assays

As described previously (Dietrich *et al.,* 2017), clonogenic assays were performed to analyze stem cell behavior, attachment dependent colony formation and growth of cancer cells.

Anchorage independent colony formation and growth of cancer cells were analyzed using soft agar colony formation assays as described previously (Borowicz *et al.,* 2014). Treatment with specific agonists and antagonists started 24 h after tumor cell seeding. Treatment was repeated after five and nine days. Microscopic analysis of the obtained colonies was conducted 14 days after tumor cell seeding.

### A.15 Analysis of cell migration and invasion

As described previously (Dietrich *et al.,* 2017), Boyden chamber assays were used in order to analyze cell migration. Boyden chamber filters were additionally coated with Matrigel to analyze cell invasion.

### A.16 NPY-ELISA

NPY-ELISA kits (murine and human) (Merck) were applied as described (Dietrich *et al.,* 2020), according to the manufacturer's instructions to quantify NPY both in cell lysates and supernatants, as well as in serum samples.

### A.17 cAMP BRET assay

A bioluminescence resonance energy transfer (BRET) assay was used to analyze cAMP signaling (Dietrich *et al.,* 2020). The biosensor CAMYEL (cAMP sensor using YFP-Epac-RLuc) served for detection of changes in intracellular cAMP concentration upon activation of Y5R30. Transfected cells were treated with 5 µM coelenterazine and intracellular cAMP level was artificially raised using 10 µM forskolin for efficient detection of the BRET signal after Y5R activation.

### A.18 Phospho-Kinase Array

A commercially available human Phospho-Kinase Array Kit (R&D Systems) was used to simultaneously determine the relative phosphorylation levels of 37 different kinase phosphorylation sites. Human melanoma cells (SkMel28) were treated for 5, 30 or 60 min with 100 nM of the Y5R agonist BWX46 and 300 µg total protein was used. The Assay was performed according to the manufacturer's instructions.

### A.19 Graphical illustration

The graphical illustrations were drawn applying the GraphPad Prism Software (GraphPad Software, Inc., San Diego, CA, USA) and SPSS (SPSS Statistics 23, IBM Corp., USA). Parts of the figures were drawn by using pictures derived from Servier Medical Art. Servier Medical Art by Servier is licensed under a Creative Commons Attribution 3.0 Unported License (https://creativecommons.org/licenses/by/3.0/).

### A.20 Study approval

In accordance with international guidelines on the ethical use of animals, the animal studies were approved (RUF-55.2.2-2532-2-566-11) by the local government's Committee for Animal Health and Care. Before enrolling in the study, patients signed an informed consent in accordance with the Helsinki Declaration. A nonprofit state-controlled foundation administers the Biobank at the Ludwig-Maximilians-Universität München (HTCR), following ethical approval (LMU Munich, No. 25-12).

### B Experiments

### B.1 EXAMPLE 1: Reduced liver metastasis in NPY^{-/-} mice is uncoupled from steatosis

Controversial data suggested that the degree of steatosis could impact melanoma and colorectal cancer (CRC) associated liver metastasis. Only mild steatosis was associated with increased liver metastasis, while the presence of NAFLD-related fibrosis was shown to reduce liver metastasis. The inventors hypothesized that NPY-associated metabolic effects might affect mild/moderate fatty change of the liver microenvironment and associated metastasis.

To test the hypothesis, a preclinical mouse model of hepatic metastases *via* hemispleen injection of syngeneic melanoma cells was applied. This model produces invasive and proliferative liver metastasis without evidence of significant immune cell infiltration, inflammation or apoptosis, which was confirmed by immunostaining for inflammation and apoptosis markers, such as Caspase-3, CD45. CD31, CD8 and CD4. This was crucial for the purpose of exploring specific steatosis-associated effects, avoiding e.g. cytokine-driven bias. To induce bland (i.e. non-inflammatory and non-fibrotic) steatosis, short-term application of a Western-type diet (WTD) was performed for time period of 6 days, using wildtype and NPY^{-/-} mice. Wildtype mice fed with a normal diet served as controls.

In wildtype mice, WTD induced weight gain and mild to moderate liver steatosis, without evidence of significant inflammatory activity or fibrosis. Unexpectedly, NPY^{-/-} mice revealed similar WTD-induced weight gain, steatosis, inflammatory activity and fibrosis, when compared to NPY^{+/+} mice. Equal effects on steatosis, inflammatory activity and fibrosis were observed in males and females. Moreover, hepatic NPY expression was not affected both by short-term (6 weeks) and chronic (6 months) WTD-induced steatosis. Likewise, in mice with high fat diet-induced steatosis and in humans with NAFLD, hepatic NPY expression was not altered compared with non-steatotic liver tissues in wildtype mice, based on comparison of immunostainings for NPY of aforementioned liver tissue samples. Furthermore, systemic NPY levels were not affected in wildtype mice after short-term WTD-treatment and were even reduced after chronic WTD-administration in mice as well as in humans with NAFLD. Together, the degree of steatosis was not altered in NPY^{-/-} mice and conversely, fatty change of the liver did also not affect hepatic NPY expression in mice and humans.

Consistent with numerous studies, the inventors found that (bland) steatosis *per se* did not significantly promote liver metastasis. However, WTD-associated liver metastasis was markedly reduced in NPY^{-/-} mice as compared with wildtype mice. Both macroscopic metastatic tumor burden as well as the number and size of liver metastasis were strongly reduced in NPY^{-/-} mice, as observed in a macroscopic classification of liver metastasis from WT mice fed with CD, WT mice fed with WTD and NPY-/- mice fed with WTD. These data suggested that the NPY-system represents a steatosis-independent driver of liver metastasis.

### B.2 EXAMPLE 2: NPY is expressed by peri-tumorous hepatocytes in the metastatic liver niche

NPY expression in the metastatic liver niche was characterized in more detail as follows:
Glycoprotein 70 (gp70) mRNA had previously been shown to be expressed by several murine cancer cell lines including the melanoma cell line B16 and the colorectal cell line CT26 but is absent in normal mouse tissues, inflamed tissues, non-transformed cell lines, and pre-cancerous lesions. Therefore, gp70 is known to represent a biomarker for high-sensitivity quantification of murine tumor burden.

The inventors found that NPY expression levels and NPY/gp70-ratios, respectively, were specifically induced in metastatic liver tissue samples as compared with non-metastatic liver tissue samples, tumorous spleen tissue samples (i.e., the primary tumor site) and non-tumorous spleen tissue samples, respectively. However, in comprehensive immunofluorescence and immunohistochemical analyses, liver-metastatic melanoma cells were found to not express NPY both in mice and in human melanoma-derived liver metastasis. Also, as compared with human epidermal melanocytes and human nevi tissues, NPY expression was found to be low or absent in melanoma cell lines and tumor tissues, respectively. In contrast, NPY was strongly expressed by peri-metastatic/microenvironment-associated hepatocytes in melanoma-derived liver metastasis in both mice and humans. Likewise, isolated primary murine and human hepatocytes were found to strongly express and secrete NPY protein, while NPY secretion was low in hepatic stellate cells and melanoma cell lines.

Beyond melanoma, low/absent NPY expression by metastatic cancer cells and strong induction of NPY in peri-metastatic hepatocytes was confirmed applying additional mouse models of colorectal cancer (CRC) cell- and organoid-derived liver metastasis as well as in liver metastases derived from CRC patients and in further types of human cancers including pancreatic cancer (PC) and breast cancer (BC). Immunofluorescence imaging of these samples showed strong signal for NPY in peri-metastatic area, compared to reduced NPY signal in are of metastases. In summary, NPY was found to be strongly induced and exclusively expressed by hepatocytes in the metastatic liver microenvironment.

### B.3 EXAMPLE 3: TGF-β induces peri-metastatic NPY expression

NPY expression in the liver microenvironment was specifically expressed by peri-tumorous hepatocytes in close proximity to the metastatic invasion front. As shown recently by the inventors, NPY expression can be induced by hepatic transforming growth factor-beta 1 (TGFβ1) (Dietrich *et al.,* 2020). Therefore, metastasis-related microenvironment activation (including TGFβ induction) might trigger close-by NPY expression.

TGFβ mRNA expression was enhanced in metastatic as compared with non-metastatic liver tissue samples. Likewise, TGFβ and NPY mRNA expression correlated significantly in liver metastatic tissues. Consistent with microenvironment-activation and associated TGFβ induction, also metastasis-related αSMA induction (Tsilimigras *et al.,* 2021; Van den Eynden *et al.,* 2013) and a significant correlation of TGFβ and αSMA expression in metastatic liver tissue samples was confirmed in the inventors' mouse model. In humans, immunohistochemistry analysis applying a tissue micro array consisting of diverse types of liver metastasis including melanoma and CRC revealed that peri-metastatic TGFβ expression patterns strongly correlated with NPY expression. Application of hepatoblast-derived liver organoids (Dietrich *et al.,* 2020) as well as primary human hepatocytes (PHH) confirmed that recombinant TGFβ is sufficient to induce NPY expression. It can be concluded that an activated liver microenvironment and TGFβ promote peri-metastatic NPY expression.

### B.4 EXAMPLE 4: NPY promotes liver colonization and chemotactic cancer cell migration

Next, the inventors aimed at exploring the functional effects of NPY on liver metastasis. The initial data suggested that NPY might drive metastatic liver colonization independent from fatty change of the liver microenvironment. To confirm this, the described model of liver metastasis was performed applying NPY^{-/-} and wildtype mice fed with a normal diet. Also, in these non-steatotic conditions, liver metastasis was strongly reduced in NPY^{-/-} mice.

In contrast to the observed induction of NPY in peri-metastatic liver tissues, systemic NPY levels were not altered in mice with liver metastasis as compared with non-metastatic controls. Likewise, patients with melanoma-derived liver metastases did not reveal enhanced systemic NPY levels compared with patients with "non-liver" metastasis and patients without distant metastasis or a control population, respectively. Strong NPY expression in peri-metastatic liver tissues and no concomitant induction of systemic NPY-levels in liver-metastatic mice and humans suggested a potential liver-directed chemogradient of NPY as described for the CXCL12-CXCR4 axis (Mortezaee, 2021). Mechanistically, recombinant NPY induced dose-dependent chemotactic migration of the inventors' metastatic melanoma mouse model cell line *in vitro.* Moreover, murine liver lysates revealed strong chemoattractive effects on these metastatic melanoma cells, while significantly reduced effects were observed applying liver lysates derived from NPY^{-/-} mice, which could be rescued by addition of recombinant NPY. In the following, comprehensive experiments were performed to confirm that NPY is sufficient to induce dose-dependent chemotactic migration and invasion applying diverse human and murine melanoma, colorectal cancer, pancreatic cancer and breast cancer cell lines. Taken together, hepatocyte-derived NPY was identified to represent a major chemoattractive ligand strongly promoting liver colonization, chemotactic migration and invasion *in vivo* and *in vitro.*

### B.5 EXAMPLE 5: Liver metastatic cancer cells strongly express NPY-5-receptor

Next, the inventors aimed at determining the responsible receptor mediating the observed metastasis-promoting effects of niche-derived NPY.

In the metastatic mouse model cell line (B16), the NPY-5-receptor (Y5R) was identified to be strongly upregulated as compared with the benign murine melanocyte cell line Melan-a. Likewise, Y5R expression was significantly upregulated in metastatic tissues compared with non-metastatic tissues in the liver of this mouse model. Immunohistochemistry and immunofluorescence analyses revealed that marked Y5R expression was preserved by liver-metastatic B16 cells *in vivo.* Strong Y5R expression in tumor tissues was confirmed applying a further melanoma mouse model (Tg(Grm1)). Analysis of human melanoma cell lines and benign melanocytes (NHEM) confirmed strong expression of Y5R mRNA and protein levels, respectively, in cancer cells *in vitro. In vivo,* strong Y5R expression also remained preserved in human melanoma cells applying a xenograft model. In melanoma patient-derived samples, Y5R was upregulated in primary and metastatic tumor tissues as compared with nevi tissues. In metastatic liver tissue samples derived from melanoma patients, Y5R was strongly expressed in the majority of patients.

Next to melanoma, Y5R mRNA and protein expression, respectively, was detected in both human and murine colorectal (CRC), pancreatic cancer (PC) and breast cancer (BC) cell lines. Strong Y5R expression was also observed in CRC tissue samples as well as in two different models of CRC-derived liver metastasis (hemispleen injection model of CRC-derived liver metastasis; orthotopic CRC-organoid-derived liver metastasis model), which confirmed marked expression of Y5R by liver-metastatic cancer cells *in vivo.* Applying the tissue micro array comprising diverse patient-derived liver metastasis, strong expression of Y5R by liver metastatic cancer cells was detected in CRC as well as in further types of cancer including PC and BC.

In line with these data, analysis of a further tissue micro array containing primary tumor tissues revealed that the emergence of liver metastasis is correlated with stronger Y5R expression as compared with non-liver-metastatic melanoma patients. Moreover, uveal and mucosal melanoma tissues which represent aggressive, high-risk subtypes for the emergence of liver metastasis were associated with stronger Y5R expression. Likewise, the emergence of liver metastasis was associated with stronger Y5R expression in CRC patient tissues as compared with non-metastatic and non-liver-metastatic-cohorts, respectively. Similar results were confirmed analyzing the complete cohort (n=1,014) comprising of melanoma (n=252), CRC (n=351), PC (n=176) and BC (n=235) tissues. Moreover, high Y5R immunohistochemistry scores correlated with a poor overall survival of CRC patients. These data were confirmed applying an additional TCGA (The Cancer Genome Atlas) derived patient dataset. It was concluded that Y5R is strongly expressed by liver-metastatic cancer cells and appeared to be a top candidate NPY-receptor potentially mediating NPY-induced liver colonization.

### B.6 EXAMPLE 6: Y5R drives NPY-induced liver colonization

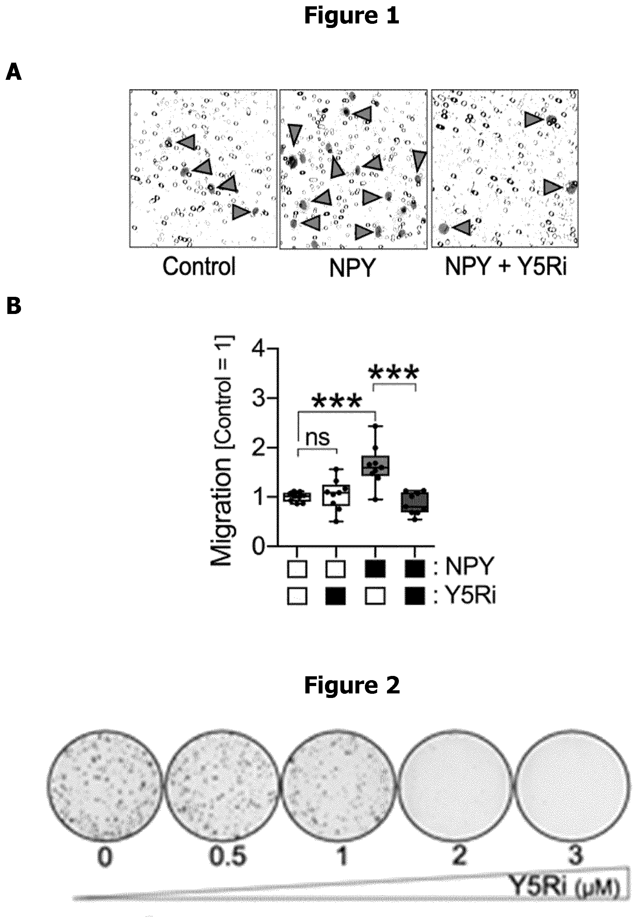

Functionally, small-molecule mediated specific inhibition of Y5R (Y5Ri) antagonized NPY-induced chemotactic migration in human melanoma cells (Figure 1A and 1B). Conversely, a specific Y5R-agonist (Dietrich *et al.,* 2020) significantly induced chemotactic migration in the same assay. Moreover, chemotactic migration induced by either hepatocyte-derived, NPY-containing supernatants or by liver lysates from wildtype mice, respectively, was significantly reduced by concomitant Y5R-inhibition. Reduction of chemotactic migration was also confirmed applying si-RNA-mediated inhibition of Y5R. Similar effects demonstrating Y5R-mediated migration were confirmed applying multiple human and murine melanoma, CRC, PC and BC cell lines as well as CRC organoids derived from a liver metastasis model.

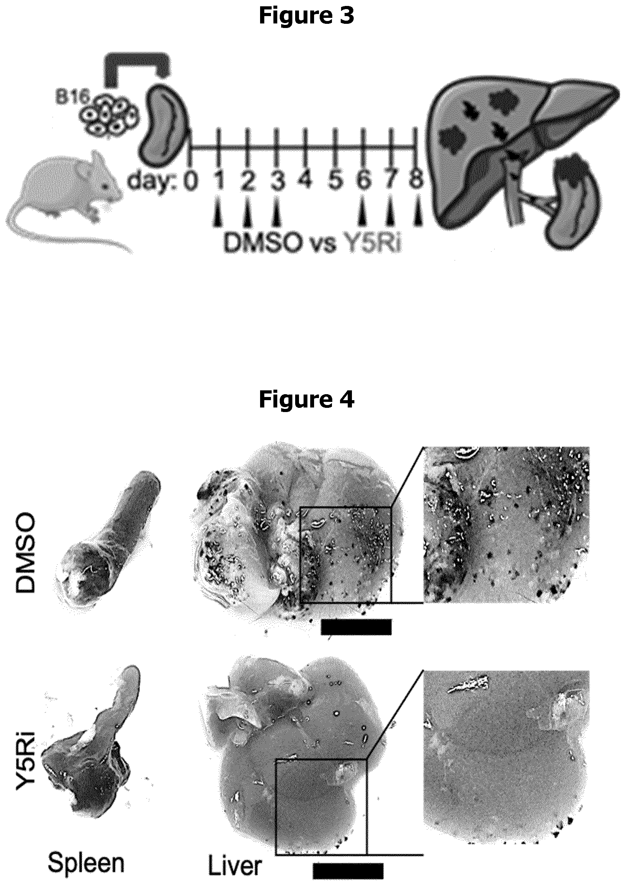

Next to migration and invasion, efficient liver metastasis requires sufficient colony formation capacities of cancer cells (Tsilimigras *et al.,* 2021; Van den Eynden *et al.,* 2013). Y5Ri strongly reduced clonogenicity of human melanoma cells applying two- and three-dimensional colony formation assays (Figure 2). These effects were confirmed applying CRC, PC and BC cell lines. *In vivo,* a therapeutic approach applying specific small molecule-mediated Y5Ri significantly reduced liver metastasis (Figures 3-5). Y5Ri did not affect liver transaminases in mice and only slightly, however not significantly, enhanced cleaved caspase 3 expression in liver metastatic cells. In contrast, Ki67 expression in liver metastasis was significantly reduced in Y5Ri-treated mice as compared with control-treated mice. Likewise, metastatic CRC organoids confirmed significantly reduced Ki67 expression after Y5Ri compared with controls. Immunohistochemical analysis of human liver metastatic tissues confirmed that high Y5R expression was correlated with enhanced Ki67 expression.

In contrast, immunohistochemical staining of CD3, CD4 and CD8 of liver metastatic tissues applying both Y5Ri-treated mice and NPY^{-/-} (as compared with the respective controls) revealed no evidence for NPY-Y5R-signaling associated effects on immune cell infiltration/inflammation. Taken together, Y5R was found to mediate NPY-induced chemotactic migration and invasion. Furthermore, Y5Ri strongly reduced colony formation, proliferation and liver colonization both *in vitro* and *in vivo.*

### B.7 EXAMPLE 7: Y5R promotes migration and clonogenicity via cAMP inhibition, ERK-activation and CHK2-dephosphorylation

Next, downstream signaling of NPY-Y5R crosstalk was analyzed. NPY potently antagonizes cAMP signaling *via* activation of Gi-proteins (Dietrich *et al.,* 2020). Bioluminescence resonance energy transfer (BRET) analysis revealed significant inhibition of forskolin-induced cAMP activation in melanoma cells by NPY and Y5R-agonist treatment. Functionally, NPY-induced enhanced chemotactic migration was restored by co-treatment applying the cAMP inductor forskolin. In line with the inventors' data, cAMP activation was confirmed to reduce migration in diverse types of (cancer) cells (Chen *et al.,* 2008). Next to cAMP inactivation, ERK activation is a strong inducer of cell motility in cancer (Vial *et al.,* 2003). Likewise, the inventors' mouse model of liver metastasis revealed marked phospho-ERK (pERK) expression with particularly prominent staining of the invading tumor edges in close proximity to peri-metastatic, NPY-expressing hepatocytes. In contrast, pERK expression in liver metastasis was strongly reduced in NPY^{-/-} compared with wildtype mice both in steatotic and non-steatotic conditions. Mechanistically, treatment of murine metastatic melanoma cells that were used in the inventors' mouse model with both NPY as well as the specific Y5R agonist was sufficient to significantly induce ERK-activation. These effects were confirmed applying human melanoma cell lines as well as human CRC cell lines and organoids. Applying the tissue micro array comprising diverse types of patient-derived liver metastasis, pERK expression was found to significantly correlate with Y5R expression. Moreover, marked correlation of Y5R and pERK expression in cancer cells expression was confirmed applying the primary tumor tissue micro array comprising large cohorts of melanoma, CRC, PC and BC patients. Functionally, Y5R-agonist-induced enhanced chemotactic migration was restored by co-treatment applying small-molecule mediated ERK inhibition. Together, NPY-Y5R crosstalk appeared to promote chemotactic migration and invasion of metastatic cancer cells *via* cAMP inactivation and ERK induction.

Next to migration/invasion, NPY-Y5R was associated with expression/proliferation of Ki67/Ki67-positive cells, as immunostainings showed reduction of Ki67-positive cells upon inhibition of Y5R. In line with this, ERK inhibition was found to reduce proliferation in liver metastatic cancer cells and pERK strongly correlated with Ki67 expression in liver metastasis in humans, while the cAMP inductor forskolin revealed no effects on proliferation.

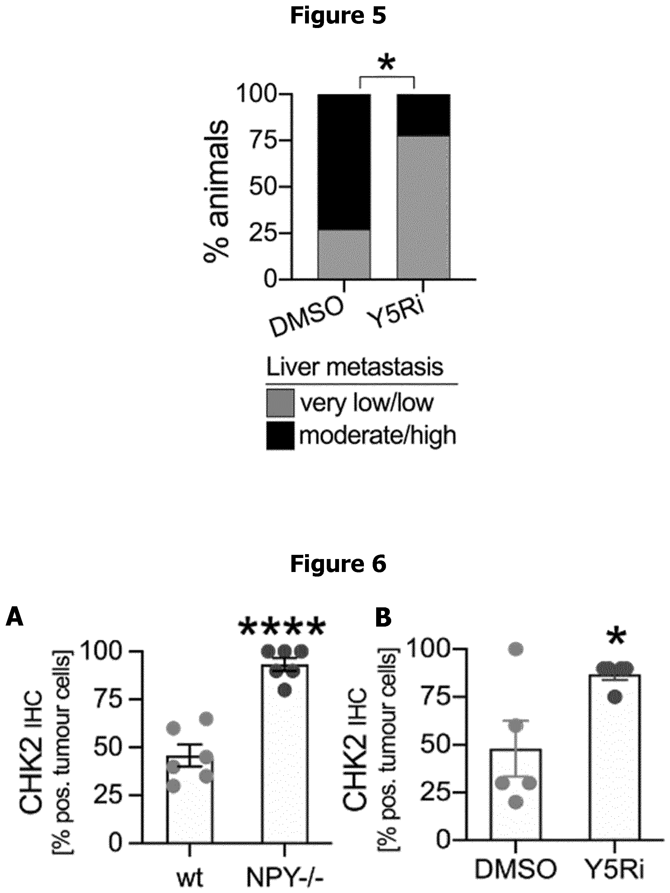

Beyond migration/invasion and proliferation, NPY-Y5R crosstalk strongly promoted colony formation (Figure 2). However, cAMP induction did not affect clonogenicity in melanoma and CRC cells, and ERK inhibition only slightly reduced clonogenicity when applying high doses of the ERK inhibitor. The inventors hypothesized that NPY-Y5R-crosstalk might affect further, yet unknown downstream effector(s) promoting clonogenicity in liver metastatic cancer cells. Subsequent multi-phospho-kinase array analysis revealed that Y5R-agonist treatment induced time-dependent dephosphorylation of checkpoint kinase 2 (CHK2) at Thr68 in human metastatic melanoma cells, as was shown by reduced phosphorylated CHK2 levels in phospho-kinase-array-based expression analysis after stimulation with Y5R-Ago for 5, 30 and 60 minutes. Activated CHK2 (pCHK2) is part of the DNA damage checkpoint pathway and represents a potent tumor suppressor. In melanoma and CRC cells, CHK2 can induce G2/M cell cycle arrest (Pan *et al.,* 2019). In breast cancer, inactivation or loss of CHK2 was shown to promote a metastatic phenotype. Y5R-agonist treatment confirmed dose-dependent reduction of pCHK2 levels in metastatic melanoma cells *in vitro.* Conversely, Y5R-inhibition induced pCHK2 in melanoma cells. These data were confirmed applying CRC organoids. In human liver metastasis, the tumor suppressor pCHK2 was poorly expressed in the majority of samples, and pCHK2 levels were inversely correlated with Y5R immunohistochemistry scores. Moreover, in the mouse models, significant induction of the tumor suppressor pCHK2 was found in NPY^{-/-} compared with wildtype mice as well as in Y5Ri-treated mice compared with controls (Figure 6A and 6B), confirming that pCHK2 is a NPY-Y5R target *in vivo.* Functionally, chemical induction of pCHK2 applying Cinobufagin (Cino) strongly reduced clonogenicity in metastatic melanoma cells. Marked reduction of clonogenic capacities of melanoma cells by Cino-mediated pCHK2 induction had been confirmed in previous studies (Pan *et al.,* 2019). It was found that co-treatment applying Cino and high doses of the Y5R agonist was sufficient to rescue Cino-mediated inhibition of clonogenicity in metastatic melanoma cells. Together, NPY-Y5R crosstalk modulates cAMP-, ERK- and CHK2-signaling to promote liver metastasis.

### B.8 EXAMPLE 8: Role of further NPY-receptors in cancer cell migration and clonogenicity

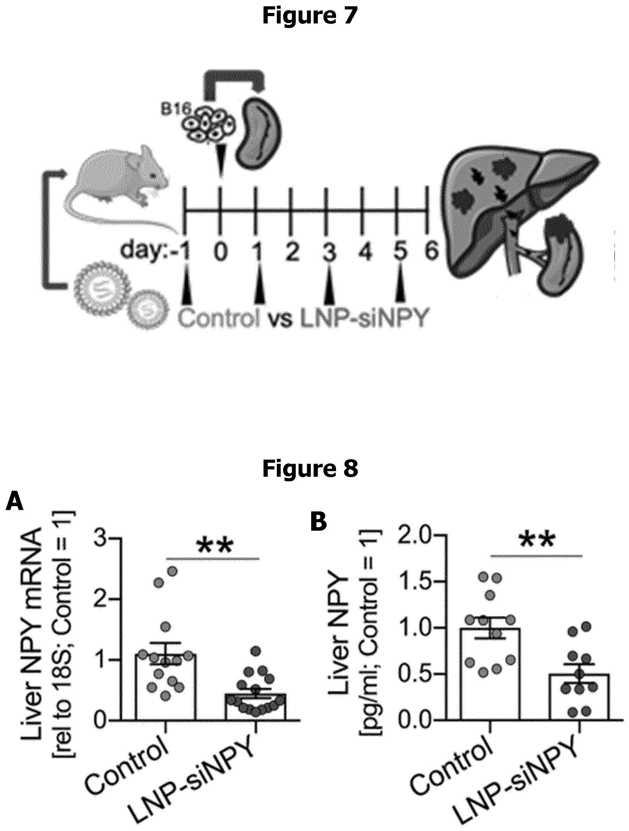

Besides Y5R, NPY can activate NPY-1 receptor (Y1R) and NPY-2 receptor (Y2R). In contrast to Y5R, Y1R and Y2R expression levels were not significantly overexpressed in metastatic as compared with non-metastatic liver tissue samples in the inventors' mouse model, which was established by comparing Y1R and Y2R mRNA levels in liver and liver metastases. Likewise, Y1R was not overexpressed in human melanoma cell lines as compared with human epidermal melanocytes, while Y2R was only slightly upregulated in melanoma cells. In metastatic melanoma cells, NPY-mediated enhanced chemotactic migration was not altered by co-treatment with specific small molecule mediated Y1R inhibition (Y1Ri) or Y2R inhibition (Y2Ri). Furthermore, clonogenicity of metastatic human melanoma cells was not affected by Y1Ri and Y2Ri, as there was no reduction in number of clones with increasing concentration of Y1R or Y2R inhibitors in clonogenicity assay. Next to human and murine melanoma cells, Y1R and Y2R was found to be expressed also by human and murine CRC, PC and BC cell lines. According to the findings in melanoma, Y1Ri and Y2Ri did not affect NPY-mediated chemotactic migration and clonogenicity in CRC cells (Figures 7A and 7B). Moreover, both Y1Ri and Y2Ri did not affect clonogenicity in PC and BC cell lines. Likewise, NPY-mediated chemotactic migration was not affected by Y2Ri in PC and BC cell lines. However, Y1Ri was sufficient to reduce NPY-mediated chemotactic migration in PC and BC cell lines.

In their mouse model, the inventors found that Y5Ri did not affect Y2R and Y5R expression levels in liver metastatic tissues, as there was no reduction in Y2R and Y5R mRNAs levels. However, NPY and Y1R expression in liver metastatic tissues as well as systemic NPY levels were upregulated upon Y5Ri treatment, as the levels of NPY and Y1R significantly increased. Moreover, while low expression of Y1R was confirmed in most human liver metastatic tissues derived from CRC, some CRC-derived liver metastasis revealed both strong Y5R and Y1R expression or even high Y1R expression, while Y5R expression was low. Likewise, analysis of TCGA-derived data revealed that next to Y5R, also Y1R (but not Y2R and NPY) were upregulated in in high-risk as compared with low-risk (based on prognostic index) CRC patient groups which was associated with a poor disease specific survival. Taken together, these findings revealed that, in contrast to Y5R, Y1R and Y2R appear not as strong promoters of migration and clonogenicity in cancer cells.

### B.9 EXAMPLE 9: RNA interference targeting the metastatic liver niche represents an effective therapeutic strategy

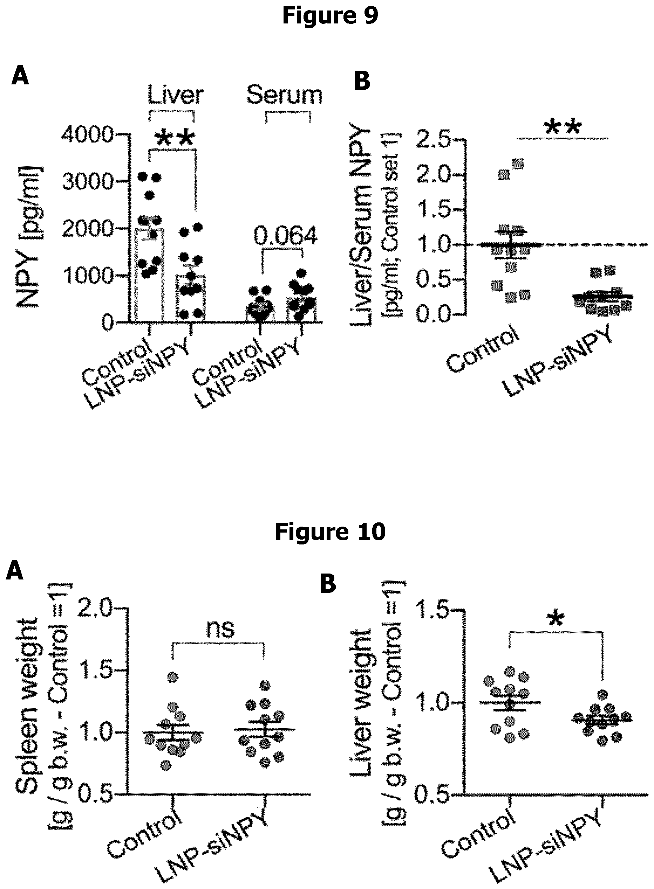

In contrast to Y5Ri-mediated induction of NPY and Y1R expression in metastatic liver tissue samples, the expression of NPY-receptors (Y1R, Y2R, Y5R) was not affected in absence of the ligand, i.e. in metastatic liver tissue samples of NPY^{-/-} mice. Since NPY is expressed predominantly by hepatocytes in close proximity of metastatic cancer cells, specific targeting of the metastatic niche rather than targeting Y5R (on cancer cells) appeared to be a particularly attractive therapeutic approach and could avoid potential resistance mechanisms. Lipid Nanoparticles (LNPs) are a promising drug delivery vehicle for clinical siRNA delivery (Evers *et al.,* 2022). The inventors designed lipid nanoparticles (LNPs) covering an siRNA pool consisting of 30 different specific siRNAs targeting murine NPY (LNP-siNPY) for a hepatocyte-specific *in vivo* knockdown (Figure 7). This therapeutic approach was sufficient to significantly reduce NPY mRNA and protein levels in liver tissues (Figures 8A and 8B). In contrast, NPY expression in "non-liver" tissues as well as systemic NPY-levels were not affected. Regarding NPY-mediated chemotactic migration, specific and significant reduction of NPY expression in liver tissues and concomitantly unaffected NPY serum levels upon LNP-siNPY treatment (Figure 9A) resulted in a strong reduction of NPY-liver-serum-ratios (Figure 9B). In contrast, body weights, spleen weights (i.e. the primary tumor site) and toxicity markers (liver transaminases and lactate dehydrogenase) were not affected upon LNP-siNPY treatment (Figure 10).

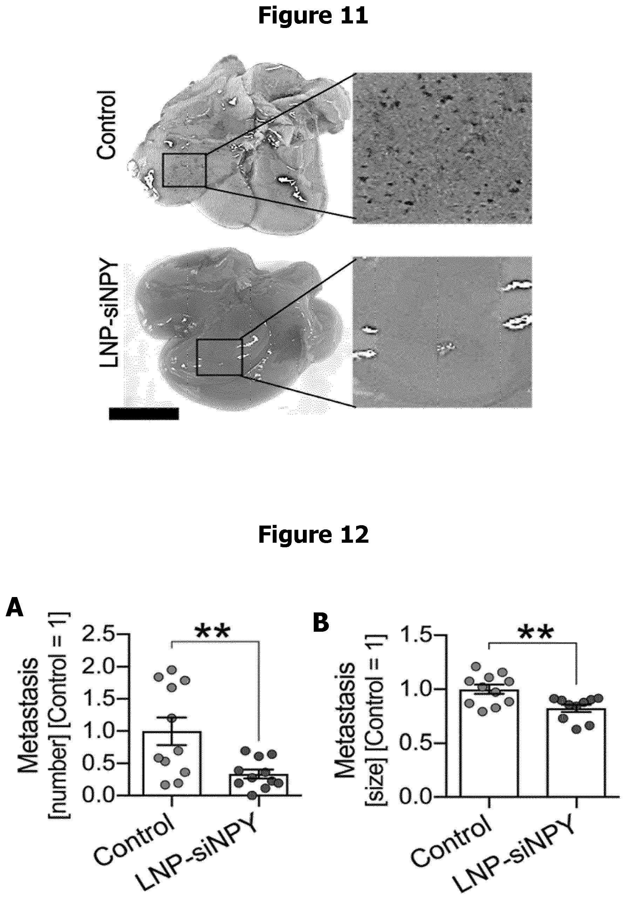

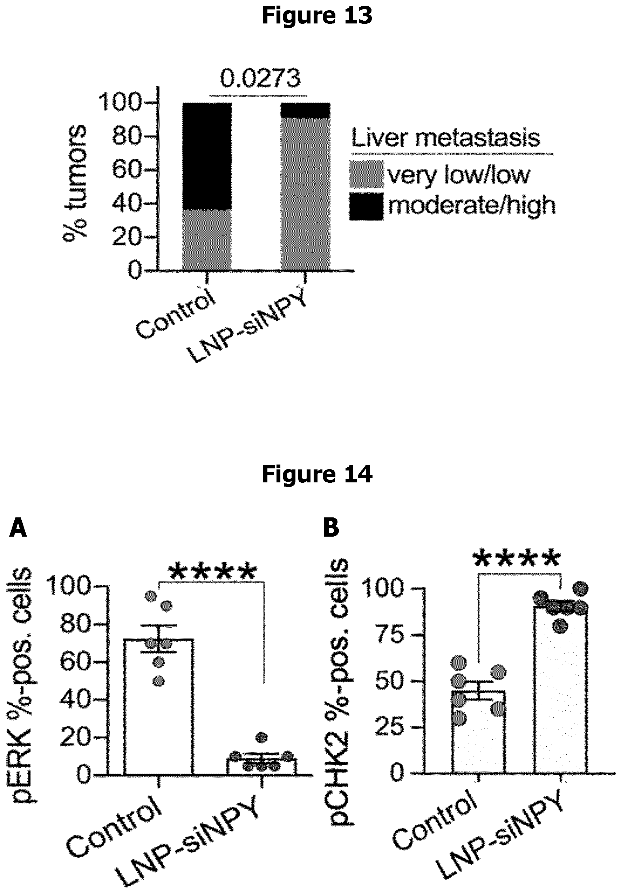

However, as compared with control treatment, LNPs bearing siNPY strongly reduced liver weights and macroscopic liver metastasis (Figure 11). Furthermore, immunohistochemistry analysis revealed that both the number and size of liver metastasis as well as Ki67-expression levels were markedly reduced in LNP-siNPY-treated mice (Figure 12 and Figure 13). Regarding NPY-Y5R-mediated downstream effectors as identified in this study, significantly reduced pERK levels as well as enhanced pCHK2 expression in liver metastasis of LNP-siNPY-treated mice confirmed the inventors' data (Figure 14). Moreover, strongly resembling the inventors' findings in NPY^{-/-} mice, targeting the ligand NPY did not induce any "compensatory" upregulation of NPY-receptors in metastatic liver tissue samples, as no changes in mRNA levels of Y1R, Y2R and Y5R in LNP-siNPY-treated mice were detected compared to the control. In summary, these findings show a novel cross-talk between niche-derived NPY and disseminated cancer cells and support a usefulness of agents targeting this cross-talk, such as RNA interference targeting niche-associated hepatocytes, in medical treatments to control liver metastasis.

### B.10 EXAMPLE 10: RNA interference targeting human NPY in primary human hepatocytes

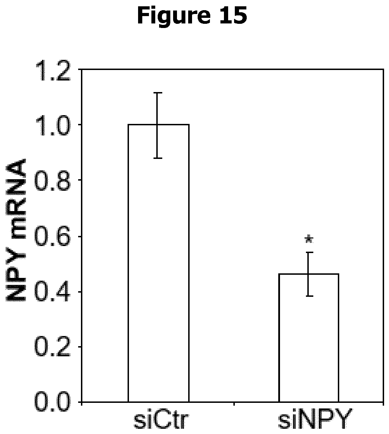

NPY mRNA expression was analyzed in primary human hepatocytes after transfection with a siRNA pool against human NPY ("siNPY") or a negative control siRNA pool ("siCtr") for 24 hours. The results confirmed that RNA interference is sufficient to significantly reduce NPY expression levels also in human primary hepatocytes (Figure 15). Moreover, additional analysis of LDH (toxicity marker) in cell supernatants after transfection of the siRNA pool against NPY as well as microscopic morphology analysis did not reveal any signs of toxicity induced by siNPY compared with the control siCTr treatment (not shown).

### REFERENCES

Adams et al. (2018). "Patisiran, an RNAi therapeutic, for hereditary transthyretin amyloidosis." New england journal of medicine, 379, 11-21.
Ahn et al. (2023). "Where should siRNAs go: applicable organs for siRNA drugs." Exp Mol Med, 55, 1283-1292.
Akinc et al. (2019). "The Onpattro story and the clinical translation of nanomedicines containing nucleic acid-based drugs." Nature nanotechnology, 14, 1084-1087.
Akinc et al. (2010). "Targeted Delivery of RNAi Therapeutics With Endogenous and Exogenous Ligand-Based Mechanisms." Molecular Therapy, 18, 1357-1364.
Balasubramaniam (1997). "Neuropeptide Y family of hormones: receptor subtypes and antagonists." Peptides, 18, 445-457.
Balwani et al. (2020). "Phase 3 Trial of RNAi Therapeutic Givosiran for Acute Intermittent Porphyria" N Engl J Med, 382, 2289-2301.
Borowicz et al. (2014). The soft agar colony formation assay. JoVE (Journal of Visualized Experiments), (92), e51998.
Cerdá-Reverter et al. (2000). "Neuropeptide Y family of peptides: structure, anatomical expression, function, and molecular evolution." Biochem Cell Biol, 78, 371-92.
Chen et al. (2008). "cAMP inhibits cell migration by interfering with Rac-induced lamellipodium formation." J Biol Chem 283, 13799-13805.
Corbett et al. (1975). "Tumor Induction Relationships in Development of Transplantable Cancers of the Colon in Mice for Chemotherapy Assays, with a Note on Carcinogen Structure" Cancer Research, 35, 2434-2439.
de Fougerolles (2008). "Delivery Vehicles for Small Interfering RNA In Vivo." Human Gene Therapy, 19, 125-132.
Dietrich etal. (2013). "Dysbalance in sympathetic neurotransmitter release and action in cirrhotic rats: impact of exogenous neuropeptide Y" J Hepatol, 58, 254-261.
Dietrich et al. (2018). "Wild type Kirsten rat sarcoma is a novel microRNA-622-regulated therapeutic target for hepatocellular carcinoma and contributes to sorafenib resistance." Gut, 67(7), 1328-1341.
Dietrich et al. (2017). Wild-type KRAS is a novel therapeutic target for melanoma contributing to primary and acquired resistance to BRAF inhibition" Oncogene, 37, 897-911.
Dietrich et al. (2020). "Molecular crosstalk between Y5 receptor and neuropeptide Y drives liver cancer. J Clin Invest 130, 2509-2526.
Dumont etal. (1992). "Neuropeptide Y and neuropeptide Y receptor subtypes in brain and peripheral tissues." Prog Neurobiol, 38, 125-67.
Evers etal. (2022). "Delivery of modified mRNA to damaged myocardium by systemic administration of lipid nanoparticles" J Control Release, 343, 207-216.
Friedrich et al. (2022). "Therapeutic siRNA: state-of-the-art and future perspectives" BioDrugs, 36(5), 549-571.
Ganesh etal. (2021). "Targeting metastatic cancer" Nat Med, 27, 34-44.
Gao et al. (2019). "Metastasis Organotropism: Redefining the Congenial Soil." Developmental Cell, 49, 375-391.
Hannus et al. (2014). "siPools: highly complex but accurately defined siRNA pools eliminate offtarget effects" Nucleic Acids Res, 42, 8049-8061.
Hartl etal. (2015). "Neuropeptide Y restores non-receptor-mediated vasoconstrictive action in superior mesenteric arteries in portal hypertension" Liver Int, 35, 2556-2563.
Heimerl et al. (2007). "Mapping ATP-binding cassette transporter gene expression profiles in melanocytes and melanoma cells" Melanoma Research, 17, 265-273.
Heichler et al. (2020). "STAT3 activation through IL-6/IL-11 in cancer-associated fibroblasts promotes colorectal tumour development and correlates with poor prognosis" Gut, 69(7), 1269-1282.
Hirsch et al. (2012). "NPY and stress 30 years later: the peripheral view" Cell Mol Neurobiol, 32, 645-659.
Hong etal. (2015). "High neuropeptide Y release associates with Ewing sarcoma bone dissemination - in vivo model of site-specific metastases" Oncotarget, 6(9), 7151-65.
Hu etal. (2020). "Therapeutic siRNA: state of the art" Signal transduction and targeted therapy, 5(1), 101.
Jacob et al. (1998). "In vitro modulation of human melanoma cell invasion and proliferation by all-trans-retinoic acid" Melanoma research, 8(3), 211-219.
Jayaraman et al. (2012). "Maximizing the potency of siRNA lipid nanoparticles for hepatic gene silencing in vivo." Angewandte Chemie (International ed. in English), 51, 8529-8533.
Judge et al. (2005). "Sequence-dependent stimulation of the mammalian innate immune response by synthetic siRNA" Nat Biotechnol, 23(4), 457-62.
Johnson et al. (1981). "Surface antigens of human melanoma cells defined by monoclonal antibodies - I. biochemical characterization of two antigens found on cell lines and fresh tumors of diverse tissue origin" Eur J Immunol, 11, 825-831.
Koch et al. (2015). "Glucose transporter isoform 1 expression enhances metastasis of malignant melanoma cells" Oncotarget, 6(32), 32748.
Krebs et al. (2017) "The EMT-activator Zeb1 is a key factor for cell plasticity and promotes metastasis in pancreatic cancer" Nature Cell Biology, 19(5), 518-529.
Lambert etal. (2017). "Emerging Biological Principles of Metastasis." Cell, 168, 670-691.
Larhammar et al. (2004). "Molecular evolution of NPY receptor subtypes." Neuropeptides, 38, 141-51.
Lee etal. (2013). "Isolation of human hepatocytes by a two-step collagenase perfusion procedure" Journal of visualized experiments (JoVE), 79, 50615.
Li et al. (2015). "Neuropeptide Y receptors: a promising target for cancer imaging and therapy." Regenerative biomaterials, 2, 215-219.
Lu et al. (2022). "Hypoxia-activated neuropeptide Y/Y5 receptor/RhoA pathway triggers chromosomal instability and bone metastasis in Ewing sarcoma" Nat Commun, 13(1), 2323.
Maestro et al. (2021). "Novel vectors and approaches for gene therapy in liver diseases." JHEP Rep, 3, 100300.
Mielgo et al. (2020). "Liver tropism in cancer: the hepatic metastatic niche" Cold Spring Harbor Perspectives in Medicine, 10, a037259.
Mortezaee (2021). "Organ tropism in solid tumor metastasis: an updated review" Future Oncol, 17(55), 1943-1961.
Pan et al. (2019). "Cinobufagin Induces Cell Cycle Arrest at the G2/M Phase and Promotes Apoptosis in Malignant Melanoma Cells" Front Oncol, 9, 853.
Reichmann et al. (2016). "Neuropeptide Y: A stressful review." Neuropeptides, 55, 99-109.
Shmushkovich etal. (2018). "Functional features defining the efficacy of cholesterol-conjugated, self-deliverable, chemically modified siRNAs" Nucleic Acids Research, 46(20), 10905-10916.
Strnad et al. (2022). "Fazirsiran for Liver Disease Associated with Alpha" N Engl J Med, 387, 514-524.
Tatemoto et al. (1982). "Neuropeptide Y-a novel brain peptide with structural similarities to peptide YY and pancreatic polypeptide." Nature, 296, 659-660.
Tsilimigras et al. (2021). "Liver metastases" Nat Rev Dis Primers, 7, 27.
Van den Eynden et al. (2013). "The multifaceted role of the microenvironment in liver metastasis: biology and clinical implications" Cancer Res, 73, 2031-2043.
Vial et al. (2003). "ERK-MAPK signaling coordinately regulates activity of Rac1 and RhoA for tumor cell motility" Cancer Cell, 4, 67-79.
Witzigmann et al. (2020). "Lipid nanoparticle technology for therapeutic gene regulation in the liver" Advanced Drug Delivery Reviews, 159, 344-363.
Woitok et al. (2020). "Lipid-encapsulated siRNA for hepatocyte-directed treatment of advanced liver disease" Cell Death & Disease, 11, 343.

## Claims

1. An inhibitor of NPY-5 receptor (Y5R) activation by hepatocyte-derived neuropeptide Y (NPY)
for use in the treatment of liver metastasis in a subject,
wherein said inhibitor (NPY/Y5R inhibitor) is selected from:
(i) at least one short interference RNA (siRNA) against NPY,
(ii) at least one Y5R inhibitor which is a small molecule,
(iii) at least one anti-NPY antibody, and
(iv) at least one anti-Y5R antibody.

2. The NPY/Y5R inhibitor for use according to claims 1, wherein the NPY/Y5R inhibitor of (i) is one siRNA against NPY, or a combination of two or more different siRNAs against NPY, particularly 2-10 different siRNAs against NPY.

3. The NPY/Y5R inhibitor for use according to claim 2, wherein the siRNA(s) comprise(s) at least one of the following chemical modifications:
(i.1.1) at least one phosphonate modification selected from methylphosphonate (MP), phosphorodithioate (PS2), methoxypropylphosphonate (MPO), 5'-phosphorothioate (5'-PS), (S)-5'-C-methyl with phosphate, peptide nucleic acid (PNA), phosphorothioate (PS) Rp isomer, phosphorothioate (PS) Sp isomer, 5'-(*E*)-vinylphosphonate (5'-(*E*)-VP), and 5'-methyl phosphonate (5'-MP);
(i.1.2) at least one ribose modification selected from 2'-O-methyl (2'OMe), 2'-deoxy-2'-fluoro (2'F), 2'-methyl-4-pyridine (2'-O-CH2Py(4)), PMO, 2'-O-methoxyethyl (2'-O-MOE), tricyclo-DNA (tcDNA), Locked Nucleic Acid (LNA), 2'-arabino-fluoro (2'-Ara-F), 2'-O-benzyl, Glycol Nucleic Acid (GNA), Unlocked Nucleic Acid (UNA), and (S)-cEt-BNA;
(i.1.3) at least one base modification selected from pseudouridine (Ψ), 2'-thiouridine (s2U), N6'-methyladenosine (m6A), N-ethylpiperidine 7'-EAA triazole modified adenine, N-ethylpiperidine 6'-triazole modified adenine, 6'-phenylpyrrolocytosine (PhpC), 5'-methylcytosine (m5C), 5'-fluoro-2'-deoxyuridine, 2',4'-difluorotoluyl ribonucleoside (rF), and 5'-nitroindole.

4. The NPY/Y5R inhibitor for use according claim 2 or claim 3, wherein the siRNA(s) is/are:
(i.2) conjugated with N-acetyl galactosamine (GalNAc); and/or
(i.3) comprised by a lipid nanoparticle (LNP), particularly an ionizable lipid nanoparticle.

5. The NPY/Y5R inhibitor for use according to any one of claims 2-4, wherein the treatment comprises injecting the siRNA(s), which are optionally conjugated with GalNAc or comprised by a LNP, into the subject, particularly by subcutaneous injection.

6. The NPY/Y5R inhibitor for use according to any of claims 2-5, wherein the siRNA(s) is/are **characterized by** one or more of the following features:
(i.4.1) each strand of the siRNA(s) has a length of 15-30 nucleotides, particularly of 19-29 nucleotides, more particularly of 19-25 nucleotides, and even more particularly of 19-24 nucleotides;
(i.4.2) a GC content of 30-60%;
(i.4.3.) asymmetry of the siRNA strands;
(i.4.4) a lack of palindromes, CCC and GGG sequences, and internal repeats.

7. The NPY/Y5R inhibitor for use according to claim 1, wherein the NPY/Y5R inhibitor is a Y5R inhibitor of (ii) selected from:
*N*[[*trans*-4-[[(4-amino-2-quinazolinyl)amino]methyl]cyclohexyl]methyl]-1-naphthalenesulfonamide (CGP71683) or a pharmaceutically acceptable salt thereof, particularly CGP71683 hydrochloride;
*trans*-N-[1-(2-fluorophenyl)-1H-pyrazol-3-yl]-1'-oxo-spiro[cyclohexane-1,3'(1'H)-furo[3,4-c]pyridine]-4-carboxamide (MK-0557);
*N-*[4-methyl-9-(1-methylethyl)-9*H*carbazol-3-yl]-4-morpholinecarboxamide (5RA972); and
*trans*-4-(tert-butylsulfonylamino)-N-[5-(trifluoromethyl)pyridin-2-yl]cyclohexane-1-carboxamide (S-2367, Velneperit).

8. The NPY/Y5R inhibitor for use according to any one of claims 1-7, wherein the treatment comprises injecting the Y5R inhibitor, particularly subcutaneously, into the subject.

9. The NPY/Y5R inhibitor for use according to any of claims 1-8, wherein the subject has or had a primary cancer, and treatment of liver metastasis is the prevention or reduction of liver metastasis by one or more of the following activities of the NPY/Y5R inhibitor in the subject:
(a) inhibiting the migration and/or invasion of cancer cells from the primary cancer into the liver of the subject;
(b) inhibiting the growth of metastatic cancer cells of the primary cancer in the liver of the subject;
(c) reducing the colony formation capacity (clonogenicity) of metastatic cancer cells of the primary cancer in the liver of the subject.

10. The NPY/Y5R inhibitor for use according to claim 9, wherein the primary cancer is selected from stomach cancer, colorectal cancer, breast cancer, pancreatic cancer, lung cancer, and malignant melanoma.

11. The NPY/Y5R inhibitor for use according to any of claims 1-9, wherein the primary cancer is different from primary liver cancer.

12. The NPY/Y5R inhibitor for use according to any of claims 1-11, wherein the subject does not have steatosis.

13. The NPY/Y5R inhibitor for use according to any of claims 1-11, wherein the subject has steatosis.

14. The NPY/Y5R inhibitor for use according to any of claims 1-13, wherein the subject is a mammal, particularly a human subject.

15. The NPY/Y5R inhibitor for use according to any of claims 1-14, wherein the treatment comprises administering the NPY/Y5R inhibitor to the subject at one, two or all of the following times:
(a) prior to treatment of a primary cancer,
(b) during treatment of a primary cancer,
(c) after treatment of a primary cancer.
